(19) Europäisches Patentamt / European Patent Office / Office européen des brevets

(11) **EP 4 585 584 A1**

(12) **EUROPEAN PATENT APPLICATION**
published in accordance with Art. 153(4) EPC

(43) Date of publication:
**16.07.2025 Bulletin 2025/29**

(21) Application number: **23863007.3**

(22) Date of filing: **28.08.2023**

(51) International Patent Classification (IPC):
***C07C 51/00*** (2006.01) ***B01J 31/24*** (2006.01)
***C07B 61/00*** (2006.01) ***C07C 53/02*** (2006.01)
***C07C 53/06*** (2006.01)

(52) Cooperative Patent Classification (CPC):
**B01J 31/24; C07B 61/00; C07C 51/00; C07C 53/02; C07C 53/06**

(86) International application number:
**PCT/JP2023/031060**

(87) International publication number:
**WO 2024/053468 (14.03.2024 Gazette 2024/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.09.2022 JP 2022141761**
**22.02.2023 JP 2023025779**

(71) Applicant: **NITTO DENKO CORPORATION**
**Ibaraki-shi**
**Osaka 567-8680 (JP)**

(72) Inventors:
- **WADA, Kazuhito**
  **Ibaraki-shi, Osaka 567-8680 (JP)**
- **HIRANO, Makoto**
  **Ibaraki-shi, Osaka 567-8680 (JP)**
- **MATSUDA, Hirokazu**
  **Ibaraki-shi, Osaka 567-8680 (JP)**
- **SERIU, Masaya**
  **Ibaraki-shi, Osaka 567-8680 (JP)**
- **PIDKO, Evgeny Alexandrovich**
  **3584HT Utrecht (NL)**
- **REBREYEND, Christophe**
  **2624BC Delft (NL)**

(74) Representative: **Hoffmann Eitle**
**Patent- und Rechtsanwälte PartmbB**
**Arabellastraße 30**
**81925 München (DE)**

(54) **CATALYTIC REACTION METHOD, METHOD FOR PRODUCING ORGANIC COMPOUND, AND CATALYST COMPOSITION**

(57) A catalytic reaction method of the present invention is a method in which a starting compound is caused to react in the presence of a solvent using a catalyst. The solvent includes an organic solvent, an aqueous solvent, and an antioxidant. The reaction of the starting compound is performed in a two-phase system in which the organic solvent and the aqueous solvent are separate. A method for producing an organic compound according to the present invention includes causing a starting compound to react in the presence of a solvent using a catalyst, to synthesize an organic compound from the starting compound. The solvent includes an organic solvent, an aqueous solvent, and an antioxidant. The reaction of the starting compound is performed in a two-phase system in which the organic solvent and the aqueous solvent are separate.

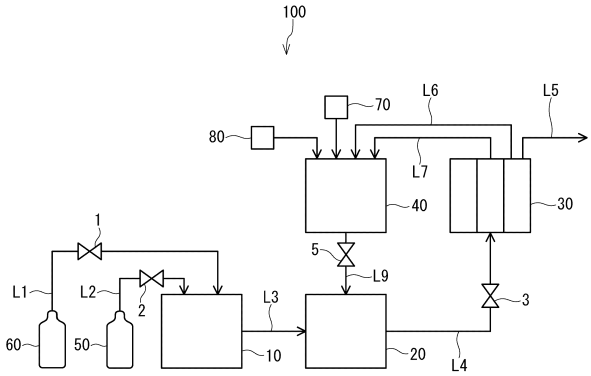


FIG.2

EP 4 585 584 A1

## Description

### TECHNICAL FIELD

The present invention relates to a catalytic reaction method, a method for producing an organic compound, and a catalyst composition.

### BACKGROUND ART

In organic synthesis reactions, various reactions in which various transition metal complexes each composed of a transition metal and ligands are used as catalysts are known.
For example, due to the problems of global warming and fossil fuel depletion, etc., there are high expectations for hydrogen energy as a next-generation energy, and methods for producing formic acid from carbon dioxide ($CO_2$) and hydrogen ($H_2$) in the presence of a catalyst are being considered.
Patent Literature 1 describes a method for producing formic acid by reaction between carbon dioxide and hydrogen in the presence of a catalyst including an element from Group 8, 9, or 10 of the periodic table, a tertiary amine (I), and a polar solvent.

### CITATION LIST

#### Patent Literature

Patent Literature 1: JP 5734286B2

### SUMMARY OF INVENTION

#### Technical Problem

However, in the conventional technology, even with a catalyst having excellent catalytic efficiency at the start of the reaction, there is a problem that the catalyst is degraded along with the progress of the catalytic reaction, resulting in a decrease in catalytic efficiency.
Therefore, an object of the present invention is to provide a catalytic reaction method, a method for producing an organic compound, and a catalyst composition that are suitable for suppressing a decrease in catalytic efficiency.

#### Solution to Problem

The present inventors have newly found, as a result of thorough examinations, that a decrease in catalytic efficiency can be suppressed by performing a reaction in a two-phase system to which an antioxidant is added, and have thus completed the present invention.
The present invention provides a catalytic reaction method including causing a starting compound to react in the presence of a solvent using a catalyst, wherein

the solvent includes an organic solvent, an aqueous solvent, and an antioxidant, and
the reaction is performed in a two-phase system in which the organic solvent and the aqueous solvent are separate.

The present invention further provides a method for producing an organic compound, including

causing a starting compound to react in the presence of a solvent using a catalyst, to synthesize an organic compound from the starting compound, wherein
the solvent includes an organic solvent, an aqueous solvent, and an antioxidant, and
the reaction is performed in a two-phase system in which the organic solvent and the aqueous solvent are separate.

The present invention further provides a catalyst composition including:

a catalyst;
an antioxidant; and
a phase-transfer catalyst.

Advantageous Effects of Invention

According to the present invention, it is possible to provide a catalytic reaction method, a method for producing an organic compound, and a catalyst composition that are suitable for suppressing a decrease in catalytic efficiency.

BRIEF DESCRIPTION OF THE DRAWINGS

FIG. 1 is a schematic diagram illustrating one example of a three-chamber type electrodialyzer.
FIG. 2 is a general schematic diagram illustrating one example of a system for producing formic acid.

DESCRIPTION OF EMBODIMENTS

A catalytic reaction method according to a first aspect of the present invention includes causing a starting compound to react in the presence of a solvent using a catalyst, wherein

the solvent includes an organic solvent, an aqueous solvent, and an antioxidant, and
the reaction is performed in a two-phase system in which the organic solvent and the aqueous solvent are separate.

According to a second aspect of the present invention, for example, in the catalytic reaction method according to the first aspect, an organic phase including the organic solvent includes the catalyst, and an aqueous phase including the aqueous solvent includes the starting compound.
According to a third aspect of the present invention, for example, in the catalytic reaction method according to the first or second aspect, an organic phase including the organic solvent includes the antioxidant.
According to a fourth aspect of the present invention, for example, in the catalytic reaction method according to any one of the first to third aspects, the antioxidant includes at least one selected from the group consisting of a phosphorus-based antioxidant, an amine-based antioxidant, and a phenolic-based antioxidant.
According to a fifth aspect of the present invention, for example, in the catalytic reaction method according to any one of the first to fourth aspects, the antioxidant includes a phosphorus-based antioxidant.
According to a sixth aspect of the present invention, for example, in the catalytic reaction method according to the fifth aspect, the phosphorus-based antioxidant is a compound represented by the following Chemical formula (1B),

[Chem. 1]

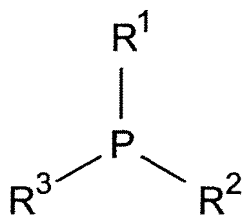

(1B)

($R^1$, $R^2$, and $R^3$ each independently represent a hydrogen atom or an optional substituent).
According to a seventh aspect of the present invention, for example, in the catalytic reaction method according to the fifth or sixth aspect, the phosphorus-based antioxidant is a compound represented by the following Chemical formula (1C),

[Chem. 2]

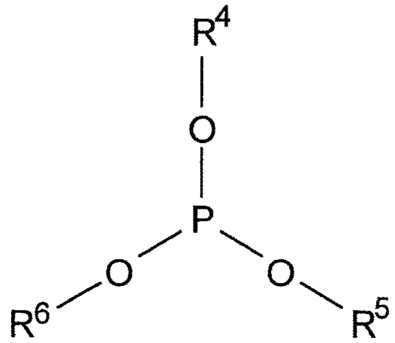

(1C)

($R^4$, $R^5$, and $R^6$ each independently represent an optional substituent).

According to an eighth aspect of the present invention, for example, in the catalytic reaction method according to the seventh aspect, in the Chemical formula (1C), $R^4$, $R^5$, and $R^6$ are each independently the following Chemical formula (1D),

[Chem. 3]

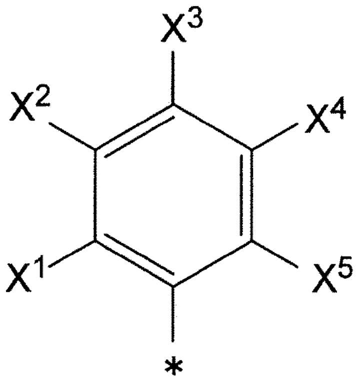

(1D)

(* represents a bond, and $X^1$, $X^2$, $X^3$, $X^4$, and $X^5$ each independently represent a hydrogen atom or a hydrocarbon group).

According to a ninth aspect of the present invention, for example, in the catalytic reaction method according to any one of the first to eighth aspects, the reaction is a hydrogenation reaction of the starting compound with hydrogen, and a hydrogenated compound of the starting compound is obtained by the reaction.

According to a tenth aspect of the present invention, for example, in the catalytic reaction method according to any one of the first to ninth aspects, the starting compound is at least one selected from the group consisting of carbon dioxide, bicarbonate, and carbonate, and a formate is obtained from the starting compound by the reaction.

According to an eleventh aspect of the present invention, for example, in the catalytic reaction method according to any one of the first to tenth aspects, the catalyst is at least one selected from the group consisting of a metal complex represented by the following General formula (1A), a tautomer of the metal complex, a stereoisomer of the metal complex, and salts thereof.

[Chem. 4]

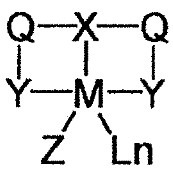

(1A)

(In General formula (1A),

X represents an atomic group including typical elements of Groups 13 to 15 that can coordinate to M,

each Q independently represents a cross-linked structure including typical elements of Groups 14 to 16 and linking Y and X,
each Y independently represents an atomic group including typical elements of Groups 14 to 16 that can coordinate to M, and M represents a metal atom,
Z represents an anionic ligand,
n represents 0 to 3, and
in a case where the number of Ls is plural, each L independently represents a neutral or anionic ligand.)

According to a twelfth aspect of the present invention, for example, in the catalytic reaction method according to the eleventh aspect, the metal complex represented by the General formula (1A) is a metal complex represented by the following General formula (2A).

[Chem. 5]

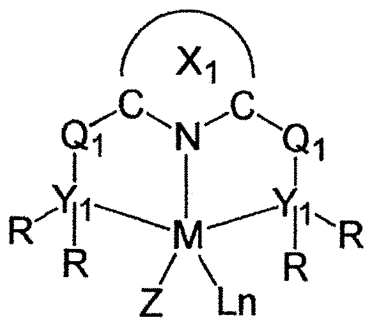

(2A)

(In General formula (2A),

$X_1$ represents a heteroaromatic ring formed with two carbon atoms and a nitrogen atom, and may have a substituent, or may combine with another substituent to form a ring,
each $Q_1$ independently represents $CH_2$, NH, or O, and $CH_2$ and NH may each further have a substituent,
each $Y_1$ independently represents a phosphorus atom or a nitrogen atom,
each R independently represents an alkyl group, an aryl group, or an aralkyl group, and may further have a substituent,
M represents a metal atom,
Z represents an anionic ligand,
n represents 0 to 3, and
in a case where the number of Ls is plural, each L independently represents a neutral or anionic ligand.)

According to a thirteenth aspect of the present invention, for example, in the catalytic reaction method according to the twelfth aspect, the metal complex represented by the General formula (2A) is a metal complex represented by the following General formula (3A).

[Chem. 6]

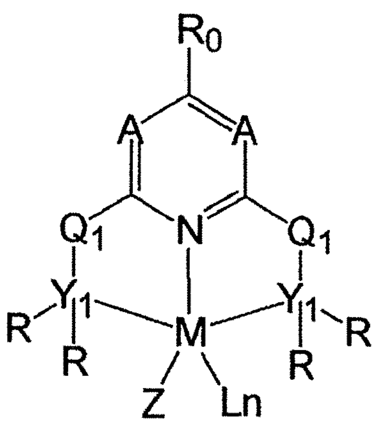

(3A)

(In General formula (3A),

$R_0$ represents a hydrogen atom or an alkyl group,
each A independently represents CH, $CR_5$, or N, and $R_5$ represents an alkyl group, an aryl group, an aralkyl group, an amino group, a hydroxy group, or an alkoxy group,
each $Q_1$ independently represents $CH_2$, NH, or O, and $CH_2$ and NH may each further have a substituent,
each $Y_1$ represents a phosphorus atom or a nitrogen atom,
each R independently represents an alkyl group, an aryl group, or an aralkyl group, and may further have a substituent,
M represents a metal atom,
Z represents an anionic ligand,
n represents 0 to 3, and
in a case where the number of Ls is plural, each L independently represents a neutral or anionic ligand.)

According to a fourteenth aspect of the present invention, for example, in the catalytic reaction method according to any one of the eleventh to thirteenth aspects, the metal atom represented by M is ruthenium.
According to a fifteenth aspect of the present invention, for example, in the catalytic reaction method according to any one of the first to fourteenth aspects, the organic solvent includes toluene.
A method for producing an organic compound according to a sixteenth aspect of the present invention includes synthesizing an organic compound from the starting compound by the catalytic reaction method according to any one of the first to fifteenth aspects.
A catalyst composition according to a seventeenth aspect of the present invention includes:

a catalyst;
an antioxidant; and
a phase-transfer catalyst.

According to an eighteenth aspect of the present invention, for example, in the catalyst composition according to the seventeenth aspect, the antioxidant includes at least one selected from the group consisting of a phosphorus-based antioxidant, an amine-based antioxidant, and a phenolic-based antioxidant.
According to a nineteenth aspect of the present invention, for example, in the catalyst composition according to the eighteenth aspect, the antioxidant includes a phosphorus-based antioxidant.
According to a twentieth aspect of the present invention, for example, in the catalyst composition according to any one of the seventeenth to nineteenth aspects, the catalyst is at least one selected from the group consisting of a metal complex represented by the following General formula (1A), a tautomer of the metal complex, a stereoisomer of the metal complex, and salts thereof,

[Chem. 7]

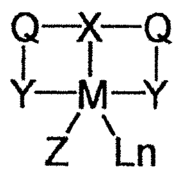

(1A)

(in General formula (1A),

X represents an atomic group including typical elements of Groups 13 to 15 that can coordinate to M,
each Q independently represents a cross-linked structure including typical elements of Groups 14 to 16 and linking Y and X,
each Y independently represents an atomic group including typical elements of Groups 14 to 16 that can coordinate to M, and M represents a metal atom,
Z represents an anionic ligand,
n represents 0 to 3, and
in a case where the number of Ls is plural, each L independently represents a neutral or anionic ligand).

According to a twenty-first aspect of the present invention, for example, the catalyst composition according to any one of the seventeenth to twentieth aspects is for generating a formate.

According to a twenty-second aspect of the present invention, for example, the catalyst composition according to any one of the seventeenth to twenty-first aspects is for a hydrogenation reaction.

According to a twenty-third aspect of the present invention, for example, the catalyst composition according to any one of the seventeenth to twenty-second aspects further includes a solvent.

According to a twenty-fourth aspect of the present invention, for example, in the catalyst composition according to the twenty-third aspect, the solvent is at least one selected from the group consisting of an organic solvent and an aqueous solvent.

The present invention will be described below in detail. However, the following description is not intended to restrict the present invention to a specific embodiment.

[Catalytic reaction method]

A catalytic reaction method according to a first embodiment of the present invention is a method in which a starting compound is caused to react in the presence of a solvent using a catalyst. The above solvent includes an organic solvent, an aqueous solvent, and an antioxidant. The reaction of the starting compound is performed in a two-phase system in which the organic solvent and the aqueous solvent are separate. In the description herein, in the two-phase system, a phase including the organic solvent may be referred to as organic phase, and a phase including the aqueous solvent may be referred to as aqueous phase. The organic phase and the aqueous phase may be collectively referred to as reaction solution.

Since the solvent includes the antioxidant, the catalyst can be inhibited from being oxidized by oxygen included in the system. Therefore, a decrease in catalytic efficiency can be suppressed. Accordingly, the catalyst can be reused, thereby reducing the production cost.

The organic phase may include the catalyst and the aqueous phase may include the starting compound. The product obtained from the catalytic reaction may be included in the aqueous phase. Accordingly, the catalyst can be easily separated, so that the catalyst can be easily reused and reactions can be repeatedly performed. In addition, the reaction in the two-phase system also has an advantage that an aqueous phase in which the concentration of the product is high can be easily produced. The organic phase may include the antioxidant. The organic phase may include the catalyst and the antioxidant, and the aqueous phase may include the starting compound.

The catalytic reaction method according to the first embodiment of the present invention is widely applicable to various reaction methods.

Examples of the methods to which the catalytic reaction method according to the first embodiment of the present invention is applicable include reduction reactions, dehydration condensation reactions, and hydrolysis reactions. The catalytic reaction method according to the embodiment of the present invention may be applied to a reduction reaction of an inorganic or organic compound. The reduction reaction is, for example, a hydrogenation reaction. That is, the catalytic reaction method according to the embodiment of the present invention may be a method in which a hydrogenated compound of the starting compound is obtained by a hydrogenation reaction of the starting compound with hydrogen. The hydrogenation reaction of an inorganic compound is, for example, a formate generation reaction. That is, the catalytic reaction method according to the embodiment of the present invention may be a method in which the starting compound is at least one selected from the group consisting of carbon dioxide, bicarbonate, and carbonate, and a formate is obtained from the starting compound. When the catalytic reaction method according to the first embodiment of the present invention is applied to a formate generation reaction, there is an advantage that it is possible to efficiently generate a formate at low cost.

(Antioxidant)

Examples of the antioxidant include primary antioxidants that capture radicals and secondary antioxidants that decompose peroxides. The antioxidant is preferably a secondary antioxidant.

Examples of the antioxidant include phosphorus-based antioxidants, amine-based antioxidants, phenolic-based antioxidants, and sulfur-based antioxidants. The antioxidant may include at least one selected from the group consisting of a phosphorus-based antioxidant, an amine-based antioxidant, and a phenolic-based antioxidant. The antioxidant may be one of the above antioxidants, or two or more of the above antioxidants may be used in combination. For example, a phosphorus-based antioxidant and a phenolic-based antioxidant may be used in combination.

The phosphorus-based antioxidant is preferably an antioxidant that is less likely to cause degradation such as hydrolysis and is highly stable, and is preferably, for example, a compound having a relatively bulky structure. The phosphorus-based antioxidant is, for example, a phosphorus compound. The phosphorus compound may be an organophosphorus compound, and may be a phosphite, a hypophosphite, or a phosphonite. Examples of the phosphite include trialkyl phosphites, triaryl phosphites, alkylaryl phosphites, and thiophosphites.

The phosphorus-based antioxidant is, for example, a compound having an aryl group. Examples of the aryl group

include C6 to C30 substituted or unsubstituted aryl groups, and the aryl group is, for example, a substituted or unsubstituted phenyl group, and is preferably a phenyl group having a t-butyl group.

The phosphorus-based antioxidant may be a compound represented by Chemical formula (1B).

[Chem. 8]

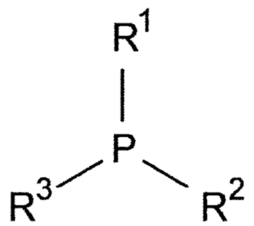

(1B)

In Chemical formula (1B), $R^1$, $R^2$, and $R^3$ each independently represent a hydrogen atom or an optional substituent. The substituent is, for example, a hydrocarbon group, a group including an oxygen atom together with a hydrocarbon, or a group including a sulfur atom together with a hydrocarbon. The number of carbon atoms in the hydrocarbon group is not particularly limited, and may be, for example, 1 to 50, 6 to 50, or even 6 to 30. The hydrocarbon group may be linear or branched. The hydrocarbon group may have a cyclic structure, or may be an aryl group. Examples of the aryl group include those described above. Examples of the hydrocarbons of the group including an oxygen atom together with the hydrocarbon and the hydrocarbons of the group including a sulfur atom together with the hydrocarbon include those described above as hydrocarbon groups. At least two selected from the group consisting of $R^1$, $R^2$, and $R^3$ may each independently have an aryl group, and $R^1$, $R^2$, and $R^3$ may each independently have an aryl group. Examples of the aryl group include those described above.

The phosphorus-based antioxidant may be a compound represented by Chemical formula (1C).

[Chem. 9]

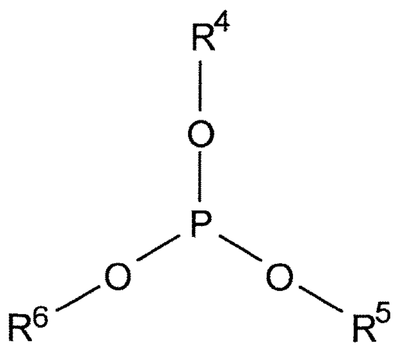

(1C)

In Chemical formula (1C), $R^4$, $R^5$, and $R^6$ each independently represent an optional substituent. Examples of the substituent include those described above. In Chemical formula (1C), $R^4$, $R^5$, and $R^6$ may each independently be the following Chemical formula (1D).

[Chem. 10]

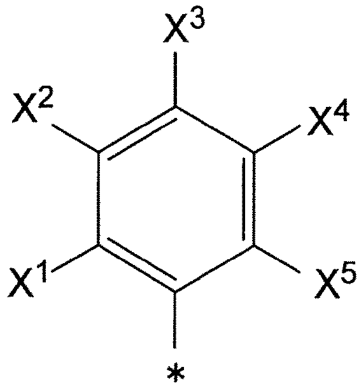

(1D)

In Chemical formula (1D), * represents a bond. $X^1$, $X^2$, $X^3$, $X^4$, and $X^5$ each independently represent a hydrogen atom or a hydrocarbon group. Examples of the hydrocarbon group include those described above. $X^1$, $X^2$, $X^3$, $X^4$, and $X^5$ may each independently be a hydrogen atom or an alkyl group, or a hydrogen atom or a tert-butyl group.

Examples of the phosphorus-based antioxidant include triphenyl phosphite, diisooctyl phosphite, heptakis triphosphite, triisodecyl phosphite, diphenyl isooctyl phosphite, diisooctyl phenyl phosphite, diphenyl tridecyl phosphite, triisooctyl phosphite, trilauryl phosphite, diphenyl phosphite, tris(dipropylene glycol)phosphite, diisodecylpentaerythritol diphosphite, dioleylhydrogen phosphite, trilauryl trithiophosphite, bis(tridecyl)phosphite, tris(isodecyl)phosphite, tris(tridecyl)phosphite, diphenyl decyl phosphite, dinonylphenyl bis(nonylphenyl)phosphite, poly(dipropylene glycol) phenyl phosphite, tetraphenyldipropylglycol diphosphite, trisnonylphenyl phosphite, tris(2,4-di-tert-butylphenyl)phosphite, tris(2,4-di-tert-butyl-5-methylphenyl)phosphite, tris[2-tert-butyl-4-(3-tert-butyl-4-hydroxy-5-methylphenylthio)-5-methylphenyl] phosphite, tridecyl phosphite, octyl diphenyl phosphite, di(decyl)monophenyl phosphite, distearyl pentaerythritol diphosphite, a mixture of distearyl pentaerythritol and calcium stearate, alkyl (C10) bisphenol A phosphite, di(tridecyl) pentaerythritol diphosphite, di(nonylphenyl)pentaerythritol diphosphite, bis(2,4-di-tert-butylphenyl)pentaerythritol diphosphite, bis(2,6-di-tert-butyl-4-methylphenyl)pentaerythritol diphosphite, bis(2,4,6-tri-tert-butylphenyl)pentaerythritol diphosphite, bis(2,4-dicumylphenyl)pentaerythritol diphosphite, tetraphenyl-tetra(tridecyl)pentaerythritol tetraphosphite, bis(2,4-di-tert-butyl-6-methylphenyl)ethyl phosphite, tetra(tridecyl)isopropylidene diphenol diphosphite, tetra(tridecyl)-4,4'-n-butylidene bis(2-tert-butyl-5-methylphenol)diphosphite, hexa(tridecyl)-1,1,3-tris(2-methyl-4-hydroxy-5-tert-butylphenyl)butane triphosphite, tetrakis(2,4-di-tert-butylphenyl)biphenylene diphosphonite, 9,10-dihydro-9-oxa-10-phosphaphenanthrene-10-oxide, (1-methyl-1-propanyl-3-ylidene)tris(1,1-dimethylethyl)-5-methyl-4,1-phenylene)hexatridecyl phosphite, 2,2'-methylene bis(4,6-di-tert-butylphenyl)-2-ethylhexyl phosphite, 2,2'-methylene bis(4,6-di-tert-butylphenyl)-octadecyl phosphite, 2,2'-ethylidene bis(4,6-di-tert-butylphenyl)fluorophosphite, 4,4'-butylidene bis(3-methyl-6-tert-butylphenyl-ditridecyl)phosphite, tris(2-[(2,4,8,10-tetrakis-tert-butyldibenzo[d,f][1,3,2]dioxaphosphepin-6-yl)oxy]ethyl)amine, 3,9-bis(4-nonylphenoxy)-2,4,8,10-tetraoxa-3,9-diphosphaspiro[5,5]undecane, 3,9-bis(2,4-di-tert-butylphenoxy)-2,4,8,10-tetraoxa-3,9-diphosphaspiro[5.5]undecane, 2,4,6-tri-tert-butylphenyl-2-butyl-2-ethyl-1,3-propanediol phosphite, poly-4,4'-isopropylidene diphenol C12-15 alcohol phosphite, and tetraalkyl(C12-15)-4,4'-isopropylidene diphenyl phosphite.

The phosphorus-based antioxidant is preferably tris(2,4-di-tert-butylphenyl)phosphite, triphenyl phosphite, triisodecyl phosphite, tetraalkyl(C12-15)-4,4',-isopropylidene diphenyl diphosphate, or 3,9-bis(2,4-di-tert-butylphenoxy)-2,4,8,10-tetraoxa-3,9-diphosphaspiro[5.5]undecane, and is particularly preferably tris(2,4-di-tert-butylphenyl) phosphite or triphenyl phosphite.

Examples of the amine-based antioxidant include 6-ethoxy-2,2,4-trimethyl-1,2-dihydroquinoline, N-phenyl-1-naphthylamine, p,p'-dioctyldiphenylamine, phenothiazine, 4,4'-bis($\alpha$,$\alpha$-dimethylbenzyl)diphenylamine, phenyl-$\alpha$-naphthylamine, phenyl-$\beta$-naphthylamine, N,N'-diphenyl-p-phenylenediamine, N,N'-di-$\beta$-naphthyl-p-phenylenediamine, N-cyclohexyl-N'-phenyl-p-phenylenediamine, N-phenyl-N'-isopropyl-p-phenylenediamine, and aldol-$\alpha$-naphthylamine.

Examples of the phenolic-based antioxidant include hindered phenolic-based antioxidants. Examples of the hindered phenolic-based antioxidants include 2,6-di-tert-butyl-p-cresol, 2,6-di-tert-butyl-4-methoxyphenol, 2-tert-butyl-4-methoxyphenol, 3-tert-butyl-4-methoxyphenol, hexadecyl 3,5-di-tert-butyl-4-hydroxybenzoate, and 2,4,6-tris(3',5'-di-tert-butyl-4'-hydroxybenzyl)mesitylene.

The phenolic-based antioxidant is not limited to the above-described hindered phenolic-based antioxidants. Examples of phenolic-based antioxidants other than the above-described hindered phenolic-based antioxidants include 2,6-di-tert-butyl-4-ethylphenol, 2-tert-butyl-4,6-dimethylphenol, styrenated phenol, 2,2'-methylene-bis(4-ethyl-6-tert-butylphenol), 2,2'-thiobis-(6-tert-butyl-4-methylphenol), 2,2'-thiodiethylene-bis[3-(3,5-di-tert-butyl-4-hydroxyphenyl)propionate], 2-methyl-4,6-bis(octylsulfanylmethyl)phenol, 2,2'-isobutylidene-bis(4,6-dimethylphenol), isooctyl-3-(3,5-di-tert-butyl-4-hydroxyphenyl)propionate, N,N'-hexane-1,6-diylbis[3-(3,5-di-tert-butyl-4-hydroxyphenyl)propionamide], 2,2'-oxamide-bis[ethyl-3-(3,5-di-tert-butyl-4-hydroxyphenyl)propionate], 2-ethylhexyl-3-(3',5'-di-tert-butyl-4'-hydroxyphenyl)propionate, 2,2'-ethylene-bis(4,6-di-tert-butylphenol), esters of 3,5-bis(1,1-dimethylethyl)-4-hydroxybenzene propanoic acid and C13-15 alkyls, 2,5-di-tert-amylhydroquinone, hindered phenol polymers (trade name AO.OH.98, manufactured by ADEKA PALMAROLE SAS), 2,2'-methylene-bis[6-(1-methylcyclohexyl)-p-cresol], 2-tert-butyl-6-(3-tert-butyl-2-hydroxy-5-methylbenzyl)-4-methylphenyl acrylate, 2-[1-(2-hydroxy-3,5-di-tert-pentylpheny)ethyl]-4,6-di-tert-pentylphenyl acrylate, 6-[3-(3-tert-butyl-4-hydroxy-5-methyl)propoxy]-2,4,8,10-tetra-tert-butylbenzo[d,f][1,3,2]-dioxaphosphepin, hexamethylene-bis[3-(3,5-di-tert-butyl-4-hydroxyphenyl)propionate], calcium bis[monoethyl(3,5-di-tert-butyl-4-hydroxybenzyl)phosphonate], a reaction product of 5,7-bis(1,1-dimethylethyl)-3-hydroxy-2(3H)-benzofuranone and o-xylene, 2,6-di-tert-butyl-4-(4,6-bis(octylthio)-1,3,5-triazin-2-ylamino)phenol, DL-a-tocopherol (vitamin E), 2,6-bis(α-methylbenzyl)-4-methylphenol, bis[3,3-bis-(4'-hydroxy-3'-tert-butyl-phenyl)butyric acid]glycol ester, 2,6-diphenyl-4-octadecyloxyphenol, stearyl(3,5-di-tert-butyl-4-hydroxyphenyl)propionate, distearyl(3,5-di-tert-butyl-4-hydroxybenzyl) phosphonate, tridecyl-3,5-tert-butyl-4-hydroxybenzyl thioacetate, thiodiethylene-bis[(3,5-di-tert-butyl-4-hydroxyphenyl) propionate], 4,4'-thiobis(6-tert-butyl-m-cresol), 2-octylthio-4,6-di(3,5-di-tert-butyl-4-hydroxyphenoxy)-s-triazine, 2,2'-methylene-bis(4-methyl-6-tert-butylphenol), bis[3,3-bis(4-hydroxy-3-tert-butylphenyl)butyric acid]glycol ester, 4,4'-butylidene-bis(2,6-di-tert-butylphenol), 4,4'-butylidene-bis(6-tert-butyl-3-methylphenol), 2,2'-ethylidene-bis(4,6-di-tert-butylphenol), 1,1,3-tris(2-methyl-4-hydroxy-5-tert-butylphenyl)butane, bis[2-tert-butyl-4-methyl-6-(2-hydroxy-3-tert-butyl-5-methylbenzyl)phenyl]terephthalate, 1,3,5-tris(2,6-dimethyl-3-hydroxy-4-tert-butylbenzyl)isocyanurate, 1,3,5-tris(3,5-di-tert-butyl-4-hydroxybenzyl)isocyanurate, 1,3,5-tris(3,5-di-tert-butyl-4-hydroxybenzyl)-2,4,6-trimethylbenzene, 1,3,5-tris [(3,5-di-tert-butyl-4-hydroxyphenyl)propionyloxyethyl] isocyanurate, tetrakis[methylene-3-(3',5'-tert-butyl-4'-hydroxyphenyl)propionate]methane, 2-tert-butyl-4-methyl-6-(2-acryloyloxy-3-tert-butyl-5-methylbenzyl)phenol, 3,9-bis[2-(3-tert-butyl-4-hydroxy-5-methylhydrocinnamoyloxy)-1,1-dimethylethyl]-2,4,8,10-tetraoxaspiro[5.5]undecane, triethylene glycol-bis[β-(3-tert-butyl-4-hydroxy-5-methylphenyl)propionate], and 3-(3,5-dialkyl-4-hydroxyphenyl)propionic acid derivatives, such as stearyl-3-(3,5-di-tert-butyl-4-hydroxyphenyl)propionic acid amide, palmityl-3-(3,5-di-tert-butyl-4-hydroxyphenyl)propionic acid amide, myristyl-3-(3,5-di-tert-butyl-4-hydroxyphenyl)propionic acid amide, and lauryl-3-(3,5-di-tert-butyl-4-hydroxyphenyl)propionic acid amide.

Examples of the sulfur-based antioxidant include dilauryl 3,3'-thiodipropionate, dimyristyl 3,3'-thiodipropionate, and distearyl 3,3'-thiodipropionate.

The antioxidant may be a phosphorus-based antioxidant. In the catalytic reaction method according to the first embodiment, in particular, the phosphorus-based antioxidant can suppress degradation of the catalyst and is particularly suitable for inhibiting a decrease in catalytic efficiency.

From the viewpoint of sufficiently exhibiting the function of the antioxidant, the amount of the antioxidant to be used is preferably 1 mmol or more with respect to 1 L of the solvent. From the viewpoint of reducing the cost of the antioxidant, the amount of the antioxidant to be used is preferably 100 mmol or less with respect to 1 L of the solvent. As the antioxidant, one kind may be used alone, or two or more kinds may be used in combination. The antioxidant may be added in its total amount when preparing a reaction solution, or the antioxidant may be added to the reaction solution in multiple portions in the middle of the reaction.

The amount of the antioxidant to be used is preferably 1 equivalent or more and 10000 equivalents or less with respect to 1 equivalent of the catalyst. The amount of the antioxidant to be used is more preferably 10 equivalents or more and 10000 equivalents or less, even more preferably 10 equivalents or more and 1000 equivalents or less, and particularly preferably 100 equivalents or more and 1000 equivalents or less.

(Catalyst)

In the catalytic reaction method according to the first embodiment of the present invention, it is preferable that at least one compound selected from the group consisting of a metal complex represented by the following General formula (1A), a tautomer of the metal complex, a stereoisomer of the metal complex, and salts thereof is used as the catalyst.

[Chem. 11]

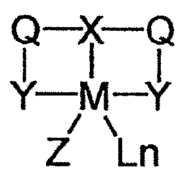

(1A)

(In General formula (1A),

X represents an atomic group including typical elements of Groups 13 to 15 that can coordinate to M,
each Q independently represents a cross-linked structure including typical elements of Groups 14 to 16 and linking Y and X,
each Y independently represents an atomic group including typical elements of Groups 14 to 16 that can coordinate to M, and M represents a metal atom,
Z represents an anionic ligand,
n represents 0 to 3, and
in a case where the number of Ls is plural, each L independently represents a neutral or anionic ligand.)

In the description herein, the "Group n" means "Group n of the periodic table".

The typical elements of Groups 13 to 15 of the periodic table in X include boron, carbon, silicon, germanium, tin, nitrogen, phosphorus, arsenic, oxygen, sulfur, and selenium atoms; boron, carbon, silicon, germanium, tin, nitrogen, phosphorus, and arsenic atoms are preferable; carbon, nitrogen, phosphorus, and sulfur atoms are more preferable; and carbon or nitrogen atom is even more preferable.

X may be an atomic group with a valence of 0 to 1. Examples of the atomic group represented by X include an alkyl group, an alkenyl group, an alkoxy group, an aromatic ring, and a heterocyclic ring, and these may each have a substituent or may each combine with another substituent to form a ring.

Examples of the alkyl group in X include linear, branched, and cyclic substituted or unsubstituted alkyl groups. The alkyl group in X is preferably a C1 to C30 alkyl group, such as a methyl group, an ethyl group, an n-propyl group, an i-propyl group, a t-butyl group, an n-octyl group, an eicosyl group, and a 2-ethylhexyl group. The alkyl group in X is preferably an alkyl group in which the number of carbon atoms is 6 or less, and is preferably a methyl group.

Examples of the alkenyl group in X include linear, branched, and cyclic substituted or unsubstituted alkenyl groups. The alkenyl group in X is preferably a C2 to C30 alkenyl group, such as a vinyl group, an n-propyl group an i-propenyl group, a t-butenyl group, and an n-octenyl group. The alkenyl group in X is preferably an alkenyl group in which the number of carbon atoms is 6 or less.

Examples of the alkoxy group in X include linear, branched, and cyclic substituted or unsubstituted alkoxy groups. The alkoxy group in X is preferably a C1 to C30 substituted or unsubstituted alkoxy group, such as a methoxy group, an ethoxy group, an isopropoxy group, a t-butoxy group, an n-octyloxy group, and a 2-methoxyethoxy group.

Examples of the aromatic ring in X include a phenyl ring and a naphthyl ring.

Examples of the heterocyclic ring in X include a pyrrolidine ring, a piperidine ring, a pyrroline ring, an imidazoline ring, an imidazolidine ring, a pyrrole ring, an imidazole ring, a pyridine ring, a pyrimidine ring, a triazine ring, a quinoline ring, and a quinazoline ring.

The atomic group with a valence of 0 to 1 represented by X preferably represents an atomic group including a heteroaromatic ring formed with two carbon atoms and a nitrogen atom, and may have a substituent, or may combine with another substituent to form a ring.

The atomic group with a valence of 0 to 1 represented by X is preferably a pyrroline ring, a pyridine ring, an imidazoline ring, a pyrimidine ring, or a triazine ring, is more preferably a pyridine ring or a triazine ring, and is even more preferably a pyridine ring.

In a case where the atomic group with a valence of 0 to 1 represented by X has a substituent, examples of the substituent include substituent group A, an alkyl group is preferable, and a methyl group is more preferable.

X may be an atomic group in which a hydrogen atom or an alkyl group is bonded to a nitrogen atom. The alkyl group is as described above, and is preferably a methyl group.

The cross-linked structure including typical elements of Groups 14 to 16 of the periodic table and linking Y and X represented by Q may have a double bond, may have a monocyclic structure or a condensed ring structure, and may have a substituent.

In Q, various structures can be introduced as described above, and the number of atoms in a portion between Y and X is, for example, preferably 1 to 5, more preferably 1 to 4, even more preferably 1 to 3, and particularly preferably 1 to 2.

The atoms in the portion between Y and X above is not particularly limited, carbon, nitrogen, phosphorus, oxygen, and sulfur atoms are preferable, carbon, nitrogen, and oxygen atoms are more preferable, carbon and oxygen atoms are even more preferable, and carbon atom is particularly preferable.

Q may have a monocyclic structure. In other words, the cross-linked structure represented by Q may include a ring structure.

In a case where Q has a monocyclic structure, the monocyclic structure may be directly bonded to Y and X in General formula (1A), or a divalent substituent may be sandwiched between the monocyclic structure and Y and/or Z in General formula (1A). Examples of the divalent substituent include a C1 to C5 alkylene group, a C2 to C5 alkenylene group,

heteroatoms such as an oxygen atom and a sulfur atom, and any of these bonded in series.

It is preferable that each Q independently represents $CH_2$, NH, or O, $CH_2$ and NH may each further have a substituent, and it is more preferable that each Q represents $CH_2$ or NH.

Q may have a condensed ring structure. In other words, the cross-linked structure represented by Q may include a condensed ring structure.

In a case where Q has a condensed ring structure, the condensed ring structure may be directly bonded to Y and X in General formula (1A), or a divalent substituent may be sandwiched between the condensed ring structure and Y and/or X in General formula (1A). The divalent substituent is the same as those described above as the divalent substituent sandwiched between the monocyclic structure and Y and/or X in General formula (1A).

Q may have a substituent. In a case where Q has neither a monocyclic structure nor a condensed ring structure, the substituent is a substituent in a Q portion in the ring structure configured to include Q, Y, X, and M in General formula (1A).

In a case where Q has a monocyclic structure or a condensed ring structure, the substituent is a substituent in the monocyclic structure or the condensed ring structure, or a substituent in another Q portion in the ring structure configured to include Q, Y, X, and M in General formula (1A).

The substituent that Q may have, for example, may have a heteroatom or may be another atom or atomic group.

Examples of the substituent having a heteroatom include a C1 to C18 alkoxy group, a C7 to C18 arylalkoxy group, a C6 to C18 aryloxy group, a C2 to C18 acyl group, a C7 to C18 aroyl group, a C2 to C18 dialkylamino group, an oxygen atom, and a sulfur atom.

Examples of the other atom or atomic group include a C3 to C18 aromatic group, a C1 to C18 alkyl group, and halogen atoms. Examples of aromatic groups include C6 to C20 aryl groups such as phenyl, xylyl, naphthyl, and biphenyl.

The number of carbon atoms in Q is preferably 12 or less, more preferably 10 or less, and even more preferably 8 or less.

Y may be an atomic group with a valence of 0 to 1. Each Y may independently represent an atomic group with a valence of 0 to 1 including typical elements of Groups 14 to 16 of the periodic table that can coordinate to M, and may further have a substituent. As the typical elements of Groups 14 to 16 of the periodic table, carbon, nitrogen, phosphorus, arsenic, oxygen, sulfur, and selenium atoms are preferable, carbon, nitrogen, phosphorus, and arsenic atoms are more preferable, nitrogen and phosphorus atoms are even more preferable, and phosphorus atom is particularly preferable.

In General formula (1A), it is preferable that each Y represents a nitrogen atom or a phosphorus atom, or that one Y represents a phosphorus atom and the other Y represents a nitrogen atom.

In a case where the atomic group with a valence of 0 to 1 represented by Y has a substituent, examples of the substituent include substituent group A, an alkyl group or an aryl group is preferable, and an ethyl group, a t-butyl group, or a phenyl group is more preferable.

M represents a metal atom. M, for example, may include elements of Groups 7 to 11 of the periodic table such as manganese, iron, ruthenium, osmium, cobalt, rhodium, iridium, nickel, palladium, platinum, copper, silver, and gold, or may include elements of Groups 8 to 11 of the periodic table such as iron, ruthenium, osmium, cobalt, rhodium, iridium, nickel, palladium, platinum, copper, silver, and gold. Among them, manganese, iron, ruthenium, cobalt, rhodium, iridium, nickel, palladium, or copper is preferable, manganese, ruthenium, rhodium, iridium, nickel, or palladium is more preferable, manganese, ruthenium, rhodium, iridium, or palladium is even more preferable, and ruthenium (Ru) is particularly preferable. M may be ruthenium (Ru) or manganese (Mn).

Examples of the anionic ligand represented by Z include halide ions (halogen atoms), a hydride ion (hydrogen atom), a nitrate ion, a cyanide ion, etc. It is preferable that Z represents a halogen atom or a hydrogen atom, and it is more preferable that Z represents a halogen atom. Z is even more preferably a chlorine atom or a bromine atom, and particularly preferably a chlorine atom.

n represents an integer from 0 to 3 and represents the number of ligands coordinated to the metal atom represented by M. From the viewpoint of catalyst stability, n is preferably 2 or 3.

In a case where the number of Ls is plural, each L independently represents a neutral or anionic ligand.

Examples of the neutral ligand represented by L include ammonia, carbon monoxide, phosphines (e.g., triphenylphosphine, tris(4-methoxyphenyl)phosphine), phosphine oxides (e.g., triphenyl phosphine oxide), sulfides (e.g., dimethyl sulfide), sulfoxides (e.g., dimethyl sulfoxide), ethers (e.g., diethyl ether), nitriles (e.g., p-methylbenzonitrile), heterocyclic compounds (e.g., pyridine, N,N-dimethyl-4-aminopyridine, tetrahydrothiophene, and tetrahydrofuran), and the like, and the neutral ligand is preferably triphenylphosphine and carbon monoxide, and is more preferably carbon monoxide.

Examples of the anionic ligand represented by L include a hydride ion (hydrogen atom), a nitrate ion, and a cyanide ion, and the anionic ligand is preferably a hydride ion (hydrogen atom).

In General formula (1A), it is preferable that X represents a heterocyclic ring, each Q represents $CH_2$, NH, or O, each Y represents a phosphorus atom, and M represents ruthenium.

It is also preferable that Z represents a chlorine atom, n represents 1 to 3, and each L independently represents a

hydrogen atom, carbon monoxide, or triphenylphosphine.

Alternatively, in General formula (1A), it is preferable that X represents an atomic group in which a hydrogen atom or an alkyl group is bonded to a nitrogen atom, each Q represents $CH_2$, NH, or O, each Y represents a phosphorus atom, and M represents manganese, and it is preferable that Z represents a bromine atom, n represents 2 to 3, and each L independently represents a hydrogen atom, carbon monoxide, or triphenylphosphine.

In the catalytic reaction method according to the first embodiment of the present invention, the metal complex represented by General formula (1A) is preferably a metal complex represented by the following General formula (2A).

[Chem. 12]

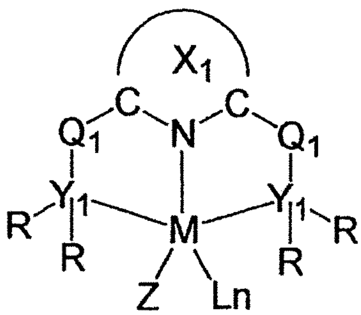

(2A)

(In General formula (2A),

$X_1$ represents a heteroaromatic ring formed with two carbon atoms and a nitrogen atom, and may have a substituent, or may combine with another substituent to form a ring,
each $Q_1$ independently represents $CH_2$, NH, or O, and $CH_2$ and NH may each further have a substituent,
each $Y_1$ independently represents a phosphorus atom or a nitrogen atom,
each R independently represents an alkyl group, an aryl group, or an aralkyl group, and may further have a substituent,
M represents a metal atom,
Z represents an anionic ligand,
n represents 0 to 3, and
in a case where the number of Ls is plural, each L independently represents a neutral or anionic ligand.)

M, $Q_1$, Z, n, and L in General formula (2A) are equivalent to M, Q, Z, n, and L in General formula (1A), respectively, and the preferable ranges are also the same.

The heteroaromatic ring formed with two carbon atoms and a nitrogen atom represented by $X_1$ is preferably a pyrroline ring, a pyridine ring, an imidazoline ring, a pyrimidine ring, or a triazine ring, is more preferably a pyridine ring or a triazine ring, and is even more preferably a pyridine ring.

Examples of the substituent that $X_1$ may have include substituent group A, an alkyl group is preferable, and a methyl group is more preferable.

Each $Y_1$ represents a phosphorus atom or a nitrogen atom, and each $Y_1$ may represent a nitrogen atom or a phosphorus atom, or one $Y_1$ may represent a phosphorus atom, and the other $Y_1$ may represent a nitrogen atom. It is preferable that each $Y_1$ is a nitrogen atom or a phosphorus atom, and it is even more preferable that each $Y_1$ is a nitrogen atom.

Examples of the alkyl group represented by R include linear, branched, and cyclic substituted or unsubstituted alkyl groups. The alkyl group represented by R is preferably a C1 to C30 alkyl group, such as a methyl group, an ethyl group, an n-propyl group, an i-propyl group, a t-butyl group, an n-octyl group, an eicosyl group, and a 2-ethylhexyl group. From the viewpoint of catalytic activity, an alkyl group in which the number of carbon atoms is 12 or less is preferable, an ethyl group or a t-butyl group is preferable, and a t-butyl group is more preferable.

The aryl group represented by R is a C6 to C30 substituted or unsubstituted aryl group, such as a phenyl group, a p-tolyl group, a naphthyl group, an m-chlorophenyl group, and an o-hexadecanoylaminophenyl group. An aryl group in which the number of carbon atoms is 12 or less is preferable, and a phenyl group is more preferable.

In a case where R further has a substituent, examples of the substituent include substituent group A, a methyl group, an ethyl group, an i-propyl group, a t-butyl group, or phenyl is preferable, and an ethyl group, an i-propyl group, or a t-butyl group is more preferable.

In General formula (2A), it is preferable that $X_1$ represents a pyridine ring or a triazine ring, each $Q_1$ represents $CH_2$, NH, or O, each $Y_1$ represents a phosphorus atom, each R represents an ethyl group, a t-butyl group, or a phenyl group, and M represents ruthenium.

It is also preferable that Z represents a chlorine atom, n represents 1 to 3, and each L independently represents a hydrogen atom, carbon monoxide, or triphenylphosphine.

In the catalytic reaction method according to the first embodiment of the present invention, the metal complex represented by General formula (2A) is preferably a metal complex represented by the following General formula (3A).

[Chem. 13]

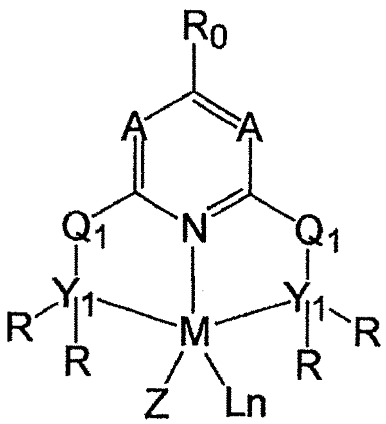

(3A)

(In General formula (3A),

$R_0$ represents a hydrogen atom or an alkyl group,
each A independently represents CH, $CR_5$, or N, and $R_5$ represents an alkyl group, an aryl group, an aralkyl group, an amino group, a hydroxy group, or an alkoxy group,
each $Q_1$ independently represents $CH_2$, NH, or O, and $CH_2$ and NH may each further have a substituent,
each $Y_1$ represents a phosphorus atom or a nitrogen atom,
each R independently represents an alkyl group, an aryl group, or an aralkyl group, and may further have a substituent,
M represents a metal atom,
Z represents an anionic ligand,
n represents 0 to 3, and
in a case where the number of Ls is plural, each L independently represents a neutral or anionic ligand.)

$Y_1$, R, $Q_1$, M, Z, n, and L in General formula (3A) are equivalent to $Y_1$, R, $Q_1$, M, Z, n, and L in General formula (2A), respectively, and the preferable ranges are also the same.

In General formula (3A), $R_0$ represents a hydrogen atom or an alkyl group. The alkyl group represented by $R_0$ is, for example, a linear, branched, or cyclic substituted or unsubstituted alkyl group. For example, the alkyl group represented by $R_0$ is preferably a C1 to C30 alkyl group, such as a methyl group, an ethyl group, an n-propyl group, an i-propyl group, a t-butyl group, an n-octyl group, an eicosyl group, and a 2-ethylhexyl group. An alkyl group in which the number of carbon atoms is 6 or less is preferable from the viewpoint of easily obtaining a raw material, and a methyl group is preferable. $R_0$ in General formula (1) preferably represents a hydrogen atom or a methyl group.

In General formula (3A), $R_0$ is preferably a hydrogen atom or a methyl group.

Each A independently represents CH, $CR_5$, or N, and $R_5$ represents an alkyl group, an aryl group, an aralkyl group, an amino group, a hydroxy group, or an alkoxy group.

The alkyl group represented by $R_5$ is, for example, a linear, branched, or cyclic substituted or unsubstituted alkyl group. The alkyl group represented by $R_5$ is preferably a C1 to C30 alkyl group, such as a methyl group, an ethyl group, an n-propyl group, an i-propyl group, a t-butyl group, an n-octyl group, an eicosyl group, and a 2-ethylhexyl group. An alkyl group in which the number of carbon atoms is 12 or less is preferable from the viewpoint of easily obtaining a raw material, and a methyl group is preferable.

The aryl group represented by $R_5$ is, for example, a C6 to C30 substituted or unsubstituted aryl group, such as a phenyl group, a p-tolyl group, a naphthyl group, an m-chlorophenyl group, or an o-hexadecanoylaminophenyl group, and an aryl group in which the number of carbon atoms is 12 or less is preferable, and a phenyl group is more preferable.

The aralkyl group represented by $R_5$ is, for example, a substituted or unsubstituted aralkyl group in which the number of carbon atoms is 30 or less, and examples thereof include a trityl group, a benzyl group, a phenethyl group, a tritylmethyl group, a diphenylmethyl group, and a naphthylmethyl group, and an aralkyl group in which the number of carbon atoms is 12 or less is preferable.

The alkoxy group represented by $R_5$ is preferably a C1 to C30 substituted or unsubstituted alkoxy group, such as a methoxy group, an ethoxy group, an isopropoxy group, a t-butoxy group, an n-octyloxy group, and a 2-methoxyethoxy group.

In General formula (3A), it is preferable that $X_1$ represents a pyridine ring or a triazine ring, each $Q_1$ represents $CH_2$, NH, or O, each $Y_1$ represents a phosphorus atom, each R represents an ethyl group, a t-butyl group, or a phenyl group, and M represents ruthenium.

It is also preferable that Z represents a chlorine atom, n represents 1 to 3, and each L independently represents a hydrogen atom, carbon monoxide, or triphenylphosphine.

In the catalytic reaction method according to the embodiment of the present invention, the metal complex represented by General formula (3A) is preferably a ruthenium complex represented by the following General formula (4A).

The ruthenium complex represented by General formula (4A) is soluble in an organic solvent and insoluble in water, and is suitable as a catalyst in the production of organic compounds. The ruthenium complex represented by General formula (4A) is suitable, for example, as a catalyst in the production of formates. Since a formate generated by a reaction is easily soluble in water, a catalyst and the formate are easily separated by a reaction in a two-phase system, and each of the catalyst and the formate is easily separated and collected from the reaction system, so that it is possible to produce a formate at a high yield and the expensive catalyst is easily reused.

[Chem. 14]

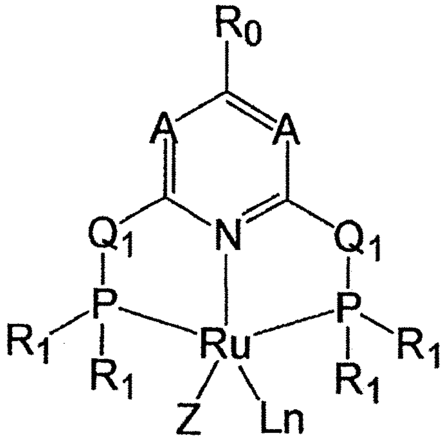

(4A)

(In General formula (4A),

$R_0$ represents a hydrogen atom or an alkyl group,
each $Q_1$ independently represents $CH_2$, NH, or O, and $CH_2$ and NH may each further have a substituent,
each $R_1$ independently represents an alkyl group or an aryl group (however, in a case where $Q_1$ represents NH or O, at least one $R_1$ represents an aryl group),
each A independently represents CH, $CR_5$, or N, and $R_5$ represents an alkyl group, an aryl group, an aralkyl group, an amino group, a hydroxy group, or an alkoxy group,
Z represents a halogen atom,
n represents 0 to 3, and
in a case where the number of Ls is plural, each L independently represents a neutral or anionic ligand.)

$R_0$, A, $Q_1$, Z, L, and n in General formula (4A) are equivalent to $R_0$, A, $Q_1$, Z, L, and n in General formula (3A), respectively, and the preferable ranges are also the same.

The alkyl group and the aryl group represented by $R_1$ are equivalent to the alkyl group and the aryl group represented by R in General formula (3A), respectively, and the preferable ranges are also the same.

As for each of the metal complexes represented by General formula (1A) to General formula (4A), stereoisomers may be generated due to a coordination form or conformation of the ligands, but the metal complex may be a mixture of these stereoisomers, or may be a pure single isomer.

As the metal complexes represented by General formula (1A) to General formula (4A), complexes produced by a known method and the like can be used. As the known method, for example, a method described in E. Pidko et al., ChemCatChem 2014, 6, 1526-1530, etc., can be used.

Specific examples of the ruthenium complex represented by General formula (4A) include compounds illustrated below. In the compounds illustrated below, Et represents an ethyl group, tBu represents a tertiary butyl group, and Ph represents a phenyl group.

[Chem. 15]

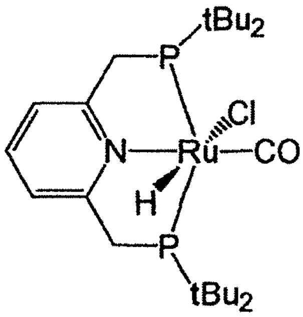

(1)

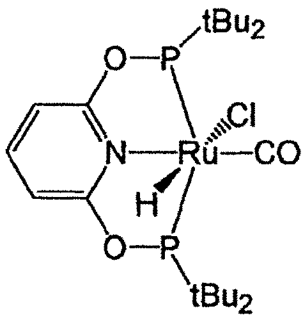

(2)

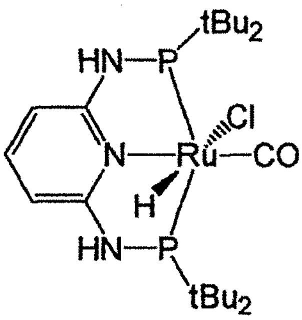

(3)

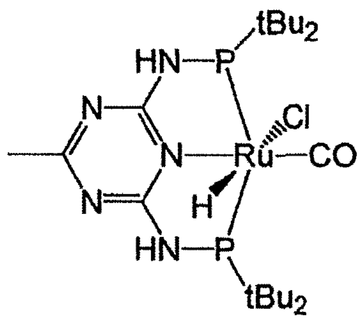

(4)

[Chem. 16]

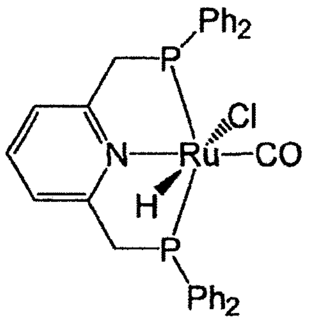

(5)

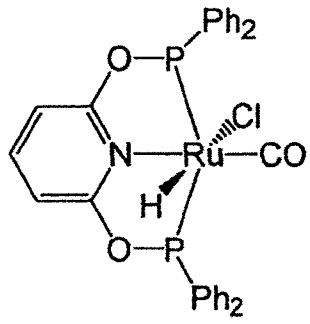

(6)

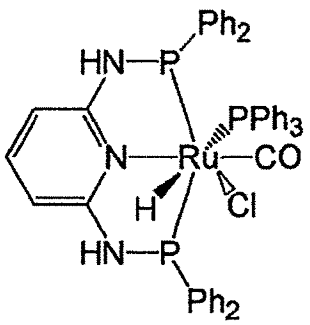

(7)

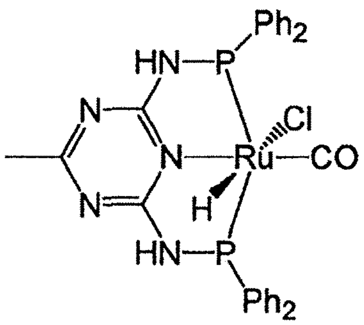

(8)

[Chem. 17]

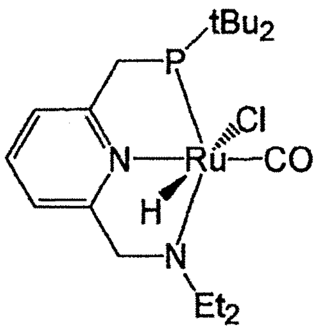

(9)

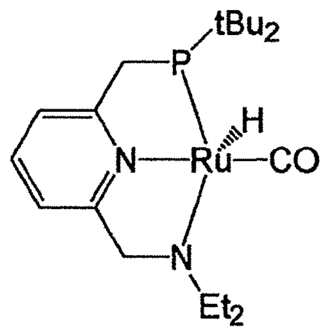

(10)

The amount of the catalyst (preferably, ruthenium complex) to be used is not particularly limited. From the viewpoint of sufficiently exhibiting the function of the catalyst, the amount of the catalyst to be used is preferably 0.1 μmol or more, more preferably 0.5 μmol or more, and even more preferably 1 μmol or more with respect to 1 L of the solvent. From the viewpoint of cost, the amount of the catalyst to be used is preferably 1 mol or less, more preferably 10 mmol or less, and even more preferably 1 mmol or less with respect to 1 L of the solvent. Furthermore, from the viewpoint of suppressing a decrease in catalytic efficiency, the amount of the catalyst to be used may be 100 μmol or less or 10 μmol or less with respect to 1 L of the solvent. In a case where two or more kinds of catalysts are used, the total amount of the catalysts to be used may be in the above-described range.

(Phase-transfer catalyst)

In the catalytic reaction method according to the first embodiment of the present invention, the reaction needs to be performed in a two-phase system. Therefore, a phase-transfer catalyst for smoothly transferring substances between the two phases may be used. Examples of the phase-transfer catalyst include a quaternary ammonium salt, a quaternary phosphate, a macrocyclic polyether such as a crown ether, a nitrogen-containing macrocyclic polyether such as a cryptand, a nitrogen-containing linear polyether, and polyethylene glycol, and an alkyl ether thereof. Among them, a quaternary ammonium salt is preferred from the viewpoint of easily transferring substances between the aqueous solvent and the organic solvent even under a mild reaction condition.

Examples of the quaternary ammonium salt include methyltrioctylammonium chloride, benzyltrimethylammonium chloride, trimethylphenylammonium bromide, tributylammonium tribromide, tetrahexylammonium hydrogen sulfate, decyltrimethylammonium bromide, diallyldimethylammonium chloride, dodecyltrimethylammonium bromide, dimethyldioctadecylammonium bromide, tetraethylammonium tetrafluoroborate, ethyltrimethylammonium iodide, tris(2-hydroxyethyl)methylammonium hydroxide, tetramethylammonium acetate, tetramethylammonium bromide, and tetraethylammonium iodide. Methyltrioctylammonium chloride is preferable.

An amount of the phase-transfer catalyst to be used is not particularly limited. The amount of the phase-transfer catalyst to be used is preferably 0.1 mmol or more, more preferably 0.5 mmol or more, and even more preferably 1 mmol or more with respect to 1 L of the organic phase and the aqueous phase solvents. The amount of the phase-transfer catalyst to be used is preferably 1 mol or less, more preferably 500 mmol or less, and even more preferably 100 mmol or less with respect to 1 L of the organic phase and the aqueous phase solvents from the viewpoint of cost. In a case where two or more kinds of phase-transfer catalysts are used, the total amount of the phase-transfer catalysts to be used may be in the above-described range.

(Solvent)

The solvent is not particularly limited as long as the solvent can form a two-phase system in which the organic solvent and the aqueous solvent are present in a separated state, and preferably includes a solvent in which the catalyst is uniformly dissolved.

Examples of the aqueous solvent include water, methanol, ethanol, ethylene glycol, glycerin, and mixed solvents thereof. Water is preferred from the viewpoint of a low load on the environment.

Examples of the organic solvent include toluene, benzene, xylene, propylene carbonate, dioxane, dimethyl sulfoxide, tetrahydrofuran, ethyl acetate, methyl cyclohexane, cyclopentyl methyl ether, and mixed solvents thereof, and the organic solvent preferably includes toluene or dioxane and more preferably includes toluene from the viewpoint of separability from the aqueous solvent. The organic solvent is, for example, toluene.

(Reaction conditions)

The reaction conditions in the catalytic reaction method according to the embodiment of the present invention are not particularly limited, and can be selected as appropriate depending on the type of reaction. The reaction conditions can also be changed as appropriate during the reaction. The form of the reaction vessel used for the reaction is not particularly limited.

The reaction temperature in the catalytic reaction method is not particularly limited, and is preferably 30°C or higher, more preferably 40°C or higher, and even more preferably 50°C or higher in order to cause the reaction to efficiently progress. The reaction temperature is preferably 200°C or lower, more preferably 150°C or lower, and even more preferably 100°C or lower from the viewpoint of energy efficiency.

The reaction temperature can be adjusted by heating or cooling, and the temperature is preferably increased by heating. For example, in the reaction between hydrogen and carbon dioxide, for example, the temperature may be increased by heating after hydrogen and carbon dioxide are introduced into the reaction vessel, or hydrogen may be introduced after carbon dioxide is introduced into the reaction vessel and the temperature is increased.

The reaction time in the catalytic reaction method is not particularly limited, and may be, for example, 0.5 hours or longer, and may be one hour or longer, two hours or longer, six hours or longer, 12 hours or longer, 24 hours or longer, 36 hours or longer, or 48 hours or longer, or even 60 hours or longer. The upper limit of the reaction time is not particularly limited, and is, for example, 500 hours or shorter, 400 hours or shorter, 300 hours or shorter, 200 hours or shorter, 100 hours or shorter, or even 80 hours or shorter.

The catalytic reaction method of the present invention is not limited to the above-described embodiment. Depending on the case, the catalytic reaction method of the present invention is not limited to a two-phase system. That is, the present invention provides a catalytic reaction method including causing a starting compound to react in the presence of a solvent using a catalyst, wherein the solvent includes an antioxidant. The solvent may include, for example, an organic

solvent.

[Method for producing organic compound]

A method for producing an organic compound according to a second embodiment of the present invention includes synthesizing an organic compound from a starting compound by the catalytic reaction method according to the first embodiment. That is, the method for producing an organic compound according to the second embodiment of the present invention includes causing a starting compound to react in the presence of a solvent using a catalyst, to synthesize an organic compound from the starting compound. The above solvent includes an organic solvent, an aqueous solvent, and an antioxidant. The reaction of the starting compound is performed in a two-phase system in which the organic solvent and the aqueous solvent are separate.

Since the solvent includes the antioxidant, the catalyst can be inhibited from being oxidized by oxygen included in the system. Therefore, a decrease in catalytic efficiency can be suppressed. Accordingly, the catalyst can be reused, which is economically advantageous.

The organic phase may include the catalyst and the aqueous phase may include the starting compound. The organic phase may include the catalyst and the aqueous phase may include the synthesized organic compound. Accordingly, the catalyst can be easily separated, so that the catalyst can be easily reused and reactions can be repeatedly performed. In addition, the reaction in the two-phase system also has an advantage that an aqueous phase in which the concentration of the synthesized organic compound is high can be easily produced. The organic phase may include the antioxidant. The organic phase may include the catalyst and the antioxidant, and the aqueous phase may include the starting compound.

The above reaction is a hydrogenation reaction of the above starting compound with hydrogen, and the above organic compound may be a hydrogenated compound of the above starting compound.

The above starting compound is at least one selected from the group consisting of carbon dioxide, bicarbonate, and carbonate, and the above organic compound may be a formate. That is, the above reaction is a hydrogenation reaction of at least one selected from the group consisting of carbon dioxide, bicarbonate, and carbonate with hydrogen, and the above organic compound may be a formate.

The antioxidant is, for example, the antioxidant described above as an antioxidant used in the catalytic reaction method according to the first embodiment. The antioxidant may include at least one selected from the group consisting of a phosphorus-based antioxidant, an amine-based antioxidant, a phenolic-based antioxidant, and a sulfur-based antioxidant. It is preferable that the antioxidant includes at least one selected from the group consisting of a phosphorus-based antioxidant, an amine-based antioxidant, and a phenolic-based antioxidant, and it is more preferable that the antioxidant includes a phosphorus-based antioxidant. The antioxidant may be a phosphorus-based antioxidant.

From the viewpoint of sufficiently exhibiting the function of the antioxidant, the amount of the antioxidant to be used is preferably 1 mmol or more with respect to 1 L of the solvent. From the viewpoint of reducing the cost of the antioxidant, the amount of the antioxidant to be used is preferably 100 mmol or less with respect to 1 L of the solvent. As the antioxidant, one kind may be used alone, or two or more kinds may be used in combination. The antioxidant may be added in its total amount when preparing a reaction solution, or the antioxidant may be added to the reaction solution in multiple portions in the middle of the reaction.

The amount of the antioxidant to be used is preferably 1 equivalent or more and 10000 equivalents or less with respect to 1 equivalent of the catalyst. The amount of the antioxidant to be used is more preferably 10 equivalents or more and 10000 equivalents or less, even more preferably 10 equivalents or more and 1000 equivalents or less, and particularly preferably 100 equivalents or more and 1000 equivalents or less.

The catalyst is, for example, the compound described above as a catalyst used in the catalytic reaction method according to the first embodiment. That is, the catalyst is, for example, at least one selected from the group consisting of the above metal complexes represented by General formulae (1A) to (4A), tautomers of the metal complexes, stereoisomers of the metal complexes, and salts thereof. The preferable ranges are also the same as in the first embodiment. In General formulae (1A) to (4A), M is preferably ruthenium.

The amount of the catalyst (preferably, ruthenium complex) to be used is not particularly limited. From the viewpoint of sufficiently exhibiting the function of the catalyst, the amount of the catalyst to be used is preferably 0.1 μmol or more, more preferably 0.5 μmol or more, and even more preferably 1 μmol or more with respect to 1 L of the solvent. From the viewpoint of cost, the amount of the catalyst to be used is preferably 1 mol or less, more preferably 10 mmol or less, and even more preferably 1 mmol or less with respect to 1 L of the solvent. Furthermore, from the viewpoint of suppressing a decrease in catalytic efficiency, the amount of the catalyst to be used may be 100 μmol or less or 10 μmol or less with respect to 1 L of the solvent. In a case where two or more kinds of catalysts are used, the total amount of the catalysts to be used may be in the above-described range.

In the production method according to the second embodiment of the present invention, the reaction needs to be performed in a two-phase system. Therefore, a phase-transfer catalyst for smoothly transferring substances between the

two phases may be used. As the phase-transfer catalyst, for example, the phase-transfer catalyst described above in the first embodiment is used. For example, in the reaction between hydrogen and at least one selected from the group consisting of carbon dioxide, bicarbonate, and carbonate, it is preferable to further use a quaternary ammonium salt as a phase-transfer catalyst. As the quaternary ammonium salt, the quaternary ammonium salt described above in the first embodiment is used, and methyltrioctylammonium chloride is preferable.

The amount of the phase-transfer catalyst to be used is not particularly limited as long as the organic compound (e.g., formate) can be produced. The amount of the phase-transfer catalyst to be used is, for example, preferably 0.1 mmol or more, more preferably 0.5 mmol or more, and even more preferably 1 mmol or more with respect to 1 L of the organic phase and the aqueous phase solvents in order for the phase-transfer catalyst to act to efficiently aid in transferring carbonate or bicarbonate. The amount of the phase-transfer catalyst to be used is preferably 1 mol or less, more preferably 500 mmol or less, and even more preferably 100 mmol or less with respect to 1 L of the organic phase and the aqueous phase solvents from the viewpoint of cost. In a case where two or more kinds of phase-transfer catalysts are used, the total amount of the phase-transfer catalysts to be used may be in the above-described range.

(Solvent)

The solvent is not particularly limited as long as the solvent can form a two-phase system in which the organic solvent and the aqueous solvent are present in a separated state, and preferably includes a solvent in which the catalyst is uniformly dissolved.

As the aqueous solvent, for example, the aqueous solvent described above in the first embodiment is used. From the viewpoint of a low load on the environment, the aqueous solvent is preferably water.

As the organic solvent, for example, the organic solvent described above in the first embodiment is used. From the viewpoint of separability from the aqueous solvent, it is preferable to include toluene or dioxane, and it is more preferable to include toluene. The organic solvent is, for example, toluene.

(Reaction conditions)

The reaction conditions in the catalytic reaction method according to the embodiment of the present invention are not particularly limited, and can also be changed as appropriate during the reaction. The form of the reaction vessel used for the reaction is not particularly limited.

The reaction temperature is not particularly limited, and is preferably 30°C or higher, more preferably 40°C or higher, and even more preferably 50°C or higher in order to cause the reaction to efficiently progress. The reaction temperature is preferably 200°C or lower, more preferably 150°C or lower, and even more preferably 100°C or lower from the viewpoint of energy efficiency.

The reaction temperature can be adjusted by heating or cooling, and the temperature is preferably increased by heating. For example, in the reaction between hydrogen and carbon dioxide, for example, the temperature may be increased by heating after hydrogen and carbon dioxide are introduced into the reaction vessel, or hydrogen may be introduced after carbon dioxide is introduced into the reaction vessel and the temperature is increased.

The reaction time is not particularly limited, and may be, for example, 0.5 hours or longer, and may be one hour or longer, two hours or longer, six hours or longer, 12 hours or longer, 24 hours or longer, 36 hours or longer, or 48 hours or longer, or even 60 hours or longer from the viewpoint of ensuring a sufficient amount of the generated organic compound and suppressing a decrease in catalytic efficiency. The upper limit of the reaction time is not particularly limited, and is, for example, 500 hours or shorter, 400 hours or shorter, 300 hours or shorter, 200 hours or shorter, 100 hours or shorter, or even 80 hours or shorter.

The production method according to the second embodiment is suitable for suppressing deterioration of catalytic efficiency. The suppression of deterioration of catalytic efficiency can be evaluated, for example, by transition of a turnover number (TON) of the catalyst when the reaction of the starting compound is repeatedly performed using the catalyst. The TON is calculated by a mathematical expression: (amount of substance of generated organic compound)/(amount of substance of catalyst used in reaction).

For example, the ratio of the TON of the catalyst in the second reaction (TON(2)) to the TON of the catalyst in the first reaction (TON(1)) is 0.7 or more, preferably 0.8 or more, more preferably 0.9 or more, and even more preferably 1.0 or more. The upper limit is not limited, and is, for example, 1.2 or less. For example, the ratio of the TON of the catalyst in the third reaction (TON(3)) to the TON(1) is 0.4 or more, preferably 0.7 or more, more preferably 0.8 or more, and even more preferably 1.0 or more. The upper limit is not limited, and is, for example, 1.2 or less. The ratio of the TON of the catalyst in the nth reaction to the TON of the catalyst in the first reaction is calculated by the mathematical expression: TON(n)/-TON(1).

In the production method according to the second embodiment, it is preferable to have a sufficient yield for practical use in the reaction of the starting compound. For example, the yield in the first reaction is 10% or more, preferably

30% or more, and more preferably 50% or more. The yield in the second reaction when the reaction of the starting compound is repeatedly performed using the catalyst is, for example, 10% or more, preferably 30% or more, and more preferably 50% or more. The yield in the third reaction is, for example, 10% or more, preferably 30% or more, and more preferably 50% or more. The upper limit of the yield is not particularly limited, and is, for example, 99% or less.

The method for producing an organic compound according to the present invention is not limited to the above-described embodiment. Depending on the case, the reaction in the method for producing an organic compound according to the present invention is not limited to a two-phase system. That is, the present invention provides a method for producing an organic compound, including causing a starting compound to react in the presence of a solvent using a catalyst, to synthesize an organic compound from the starting compound, wherein the solvent includes an antioxidant. The solvent may include, for example, an organic solvent.

The method for producing an organic compound according to the present invention may be a batch type or a continuous type. In the continuous type, for example, the reaction of the starting compound is performed using a continuous stirred tank reactor (CSTR) or a plug flow reactor (PFR).

Hereinafter, a case where the organic compound is a formate in the method for producing an organic compound according to the second embodiment of the present invention, that is, a case of a method for producing a formate, will be described in detail.

(Method for producing formate)

The method for producing a formate includes, for example, a step of causing hydrogen and at least one selected from the group consisting of carbon dioxide, bicarbonate, and carbonate to react with each other in the presence of a solvent to which an antioxidant is added, using a catalyst, to generate a formate in the reaction solution. In the description herein, this step may be referred to as first step. In the first step, as described above, the solvent includes the antioxidant. Accordingly, the catalyst can be inhibited from being oxidized by oxygen included in the system. By inhibiting the oxidation of the catalyst, a decrease in catalytic efficiency can be suppressed. Accordingly, the catalyst can be reused without degradation. In the first step, hydrogen and at least one selected from the group consisting of carbon dioxide, bicarbonate, and carbonate are caused to react with each other in a two-phase system in which the organic solvent and the aqueous solvent are separate, as described above. In this reaction, the catalyst and the antioxidant are, for example, dissolved in the organic phase. The formate generated by the reaction is dissolved in the aqueous phase. Thus, the reaction for generating the formate can be inhibited from halting due to equilibrium, and the formate can be generated at a high yield. Furthermore, the aqueous phase and the organic phase can be separated by a simple method. Therefore, an expensive metal catalyst tends to be able to be reused without deactivating catalytic activity. By reusing the catalyst, high productivity can be achieved.

In the first step, hydrogen and carbon dioxide can be stored as formate (for example, alkali metal formate). Formate has a high hydrogen storage density, is safe, and stable as a chemical substance, so that formate can be easily handled, and hydrogen and carbon dioxide can be advantageously stored for a long time period. Formate has high solubility with respect to an aqueous solvent, and can be fractionated as an aqueous solution of formate which has a high concentration. After the concentration of formate is adjusted as necessary, the aqueous solution of the formate can be supplied to a formic acid producing step described below.

For example, the first step can be performed as follows. Firstly, a reaction vessel having a stirring device is prepared, and a solvent is introduced into the reaction vessel. A phase-transfer catalyst may be further added as necessary. The catalyst is added into the reaction vessel, and dissolved in the solvent to prepare a catalyst solution. The antioxidant is added to the catalyst solution in the reaction vessel. Then, hydrogen and at least one selected from the group consisting of carbon dioxide, bicarbonate, and carbonate are introduced into the reaction vessel, and the reaction is performed. The order of addition of the catalyst and addition of the antioxidant into the reaction vessel is not particularly limited. The antioxidant may be added in its total amount when preparing a reaction solution, or may be added to the reaction solution in multiple portions in the middle of the reaction.

The reaction conditions in the method for producing a formate (reaction conditions in the first step) are not particularly limited, and depending on the case, the reaction conditions may be changed as appropriate during the reaction, but it is preferable that the reaction conditions are not changed. The form of the reaction vessel used for the reaction is not particularly limited.

In the first step, for example, the reaction solution is stirred. The condition for stirring the reaction solution is not particularly limited. However, the stirring power is preferably 0.2 $kW/m^3$ or more and more preferably 0.5 $kW/m^3$ or more. The higher the stirring power is, the higher the dispersibility of gas into the aqueous phase and the organic phase tends to become. By stirring the reaction solution, gas (for example, hydrogen in the form of gas) is involved into the reaction solution from the upper portion of the liquid surface of the reaction solution, so that gas is filled into the aqueous phase and the organic phase. However, the method for filling gas into the aqueous phase and the organic phase is not limited to the above-described method, and a sparger may be used.

The shape of the stirring blade used for stirring the reaction solution is not particularly limited. Examples of the stirring blade include not only an anchor blade, a turbine blade, and a paddle blade but also a blade that is generically called large blade, such as FULLZONE (registered trademark) blade (Kobelco Eco-Solutions Co., Ltd.) and MAXBLEND (registered trademark) blade (Sumitomo Heavy Industries Process Equipment Co., Ltd.).

In the method for producing a formate, examples of the reaction include a reaction between hydrogen and carbon dioxide, a reaction between hydrogen and bicarbonate, and a reaction between hydrogen and carbonate. In the reaction between hydrogen and carbon dioxide, for example, a reaction in which carbon dioxide forms carbonate, and a reaction in which formate is generated by the carbonate and hydrogen simultaneously progress.

The method and order of introducing hydrogen and at least one selected from the group consisting of carbon dioxide, bicarbonate, and carbonate into the reaction vessel is not particularly limited. For example, in the reaction between hydrogen and carbon dioxide, hydrogen and carbon dioxide are preferably introduced simultaneously. Hydrogen and carbon dioxide may be individually introduced, or may be introduced as mixed gas. One or both of hydrogen and carbon dioxide may be continuously introduced or intermittently introduced. In the reaction between hydrogen and bicarbonate and the reaction between hydrogen and carbonate, hydrogen is preferably introduced after bicarbonate or carbonate is introduced into the reaction vessel. One or both of hydrogen, and bicarbonate or carbonate may be continuously introduced or intermittently introduced.

The reaction temperature in the reaction between hydrogen and carbon dioxide, bicarbonate, or carbonate is not particularly limited, and is preferably 30°C or higher, more preferably 40°C or higher, and even more preferably 50°C or higher in order to cause the reaction to efficiently progress. The reaction temperature is preferably 200°C or lower, more preferably 150°C or lower, and even more preferably 100°C or lower from the viewpoint of energy efficiency. The reaction temperature can be adjusted by heating or cooling, and the temperature is preferably increased by heating. In the reaction between hydrogen and carbon dioxide, for example, the temperature may be increased by heating after hydrogen and carbon dioxide are introduced into the reaction vessel, or carbon dioxide is introduced into the reaction vessel and the temperature is increased, and thereafter, hydrogen may be introduced. In the reaction between hydrogen and bicarbonate or carbonate, for example, it is preferable that after bicarbonate or carbonate is introduced (generated) into the reaction vessel, hydrogen is introduced and the temperature is increased.

In the reaction between hydrogen and at least one selected from the group consisting of carbon dioxide, bicarbonate, and carbonate, the reaction pressure (pressure of gas in the reaction vessel) is not particularly limited, and is, for example, 0.1 MPa or more, and may be 0.2 MPa or more, 0.5 MPa or more, 1 MPa or more, 4 MPa or more, 4.5 MPa or more, or even 5 MPa or more from the viewpoint of improving the TON of the metal catalyst. The upper limit value of the reaction pressure is, but is not particularly limited to, for example, 50 MPa, and may be 20 MPa or 10 MPa.

A concentration (concentration of formate in the aqueous phase) of the formate generated in the first step is preferably 1 mol/L or more, more preferably 2.5 mol/L or more, and even more preferably 5 mol/L or more in order to produce formate at a high yield with excellent productivity. The concentration of formate is preferably 30 mol/L or less, more preferably 25 mol/L or less, and even more preferably 20 mol/L or less in order to simplify the production process by producing formate in a state where the formate is dissolved.

(Carbon dioxide and hydrogen)

As hydrogen used in the present embodiment, either hydrogen in the form of gas from a gas cylinder or liquid hydrogen can be used. As a hydrogen supply source, for example, hydrogen generated in a smelting process in iron manufacture or hydrogen generated in a soda manufacturing process can be used. Hydrogen generated by electrolysis of water can also be utilized.

Carbon dioxide used in the present embodiment may be pure carbon dioxide gas, or may be a mixture with a component other than carbon dioxide. Mixed gas with another component may be prepared by individually introducing carbon dioxide gas and another gas, or mixed gas may be prepared in advance before being introduced. Examples of the component other than carbon dioxide include inert gases such as nitrogen and argon, water vapor, and any other component included in exhaust gas and the like. Examples of carbon dioxide include carbon dioxide in the form of gas from a gas cylinder, carbon dioxide in the form of liquid, supercritical carbon dioxide, and dry ice.

The hydrogen gas and the carbon dioxide gas may be individually introduced into the reaction system, or may be introduced as mixed gas. Hydrogen and carbon dioxide may be used at the same proportion on a mole basis. However, hydrogen is preferably used in excess.

In a case where hydrogen in the form of gas from a gas cylinder is used as hydrogen, the pressure of the hydrogen is, for example, 0.1 MPa or more, may be 0.2 MPa or more, 0.5 MPa or more, 1 MPa or more, 4 MPa or more, or 4.5 MPa or more, and furthermore, may be 5 MPa or more, from the viewpoint of sufficiently ensuring reactivity. The pressure of the hydrogen is preferably 50 MPa or less, more preferably 20 MPa or less, and even more preferably 10 MPa or less in order to address enlargement of facilities.

The pressure of the carbon dioxide is preferably 0.1 MPa or more, more preferably 0.2 MPa or more, and even

more preferably 0.5 MPa or more from the viewpoint of sufficiently ensuring reactivity. The pressure of the carbon dioxide is preferably 50 MPa or less, more preferably 20 MPa or less, and even more preferably 10 MPa or less in order to address enlargement of facilities.

Hydrogen gas and carbon dioxide gas may be introduced into the catalyst solution by bubbling (blowing). After hydrogen gas and gas including carbon dioxide are introduced, the catalyst solution, and hydrogen gas and carbon dioxide gas may be stirred by, for example, a stirring device or rotating a reaction vessel.

A method for introducing carbon dioxide, hydrogen, the metal catalyst, the solvent, and the like which are used for the reaction, into a reaction vessel, is not particularly limited. All of the raw materials and the like may be collectively introduced, a part or all of the raw materials and the like may be introduced stepwise, or a part or all of the raw materials and the like may be continuously introduced. An introduction method in which these methods are combined may be used.

(Bicarbonate and carbonate)

Examples of bicarbonate and carbonate used in the present embodiment include carbonate and bicarbonate of an alkali metal or an alkaline-earth metal. Examples of bicarbonate include sodium bicarbonate and potassium bicarbonate. Potassium bicarbonate is preferable from the viewpoint of high solubility with respect to water. That is, in the present embodiment, the starting compound preferably includes potassium bicarbonate as bicarbonate. Examples of carbonate include sodium carbonate, potassium carbonate, potassium sodium carbonate, and sodium sesquicarbonate.

Bicarbonate and carbonate can be generated by reaction between a base and carbon dioxide. For example, bicarbonate or carbonate may be generated by introducing carbon dioxide into a basic solution.

Examples of a solvent for the basic solution in generation of bicarbonate or carbonate include, but are not particularly limited to, water, methanol, ethanol, N,N-dimethylformamide, dimethyl sulfoxide, tetrahydrofuran, benzene, toluene, and mixed solvents thereof, and the solvent preferably includes water, and is more preferably water. The base used for the basic solution is not particularly limited as long as the base can react with carbon dioxide to generate bicarbonate or carbonate, and the base is preferably hydroxide. Examples of the base include lithium bicarbonate, sodium bicarbonate, potassium bicarbonate, cesium bicarbonate, potassium hydroxide, sodium hydroxide, diazabicycloundecene, and triethylamine. Among them, the base is preferably hydroxide, more preferably potassium hydroxide or sodium hydroxide, and even more preferably potassium hydroxide.

A content of the base in the basic solution is not particularly limited as long as bicarbonate and carbonate can be produced. The content of the base is preferably 0.1 mol or more, more preferably 0.5 mol or more, and even more preferably 1 mol or more with respect to 1 L of the aqueous phase solvent from the viewpoint of ensuring an amount of generated formate. The content of the base is preferably 30 mol or less, more preferably 20 mol or less, and even more preferably 15 mol or less from the viewpoint of efficiency of the reaction. If the content of the base is greater relative to the solubility of the aqueous phase, the solution may be suspended.

A ratio between carbon dioxide and the base in terms of amount to be used in the reaction between carbon dioxide and the base is preferably 0.1 or more, more preferably 0.5 or more, and even more preferably 1.0 or more as a molar ratio from the viewpoint of generating carbonate from carbon dioxide. The ratio is preferably 8.0 or less, more preferably 5.0 or less, and even more preferably 3.0 or less from the viewpoint of utilization efficiency of carbon dioxide. The ratio between carbon dioxide and the base in terms of amount to be used may be a ratio between molar amounts of carbon dioxide and the base to be introduced into a reaction vessel, and is a molar amount (mol) of $CO_2$/a molar amount (mol) of the base. In a case where the ratio between carbon dioxide and the base in terms of amount to be used is in the above-described range, carbon dioxide is inhibited from being excessively charged into the reaction vessel, and an amount of unreacted carbon dioxide can be minimized, so that the final formic acid conversion efficiency is likely to be enhanced. In the same vessel, carbon dioxide can be hydrogenated through bicarbonate or carbonate by reaction between carbon dioxide and the base to generate formate. Unreacted carbon dioxide can be collected from the reaction vessel and reused.

A method and an order for introducing carbon dioxide and the base into the reaction vessel are not particularly limited. However, carbon dioxide is preferably introduced after the base is introduced into the reaction vessel. One or both of carbon dioxide and the base may be continuously introduced or intermittently introduced.

In the reaction for generating bicarbonate or carbonate by reaction between carbon dioxide and the base, the reaction temperature is, but is not particularly limited to, preferably 0°C or higher, more preferably 10°C or higher, and even more preferably 20°C or higher in order to dissolve carbon dioxide in the aqueous phase. The reaction temperature is preferably 100°C or lower, more preferably 80°C or lower, and even more preferably 40°C or lower.

In the reaction for generating bicarbonate or carbonate by reaction between carbon dioxide and the base, a reaction time is, but is not particularly limited to, for example, preferably 0.5 hours or longer, more preferably one hour or longer, and even more preferably two hours or longer, from the viewpoint of sufficiently ensuring an amount of generated bicarbonate or carbonate. The reaction time is preferably 24 hours or less, more preferably 12 hours or less, and even more preferably 6 hours or less from the viewpoint of cost.

Bicarbonate and carbonate generated by reaction between carbon dioxide and the base can be used for the

reaction between hydrogen and bicarbonate or carbonate in the method for producing an organic compound according to the second embodiment of the present invention. By generating bicarbonate or carbonate by reaction between carbon dioxide and the base in the reaction vessel, bicarbonate or carbonate may be introduced into the reaction vessel.

[Method for producing formic acid]

The method for producing formic acid according to the present embodiment includes a step of producing the formate by the above-described method for producing the formate, and a step of protonating at least a part of the formate to generate formic acid. In the description herein, the step of protonating at least a part of formate to generate formic acid may be referred to as second step. The method for producing formic acid according to the present embodiment includes, for example, the above-described first step and the second step.

In the first step, since the generated formate is eluted into the aqueous phase, the aqueous solution of formate is obtained by fractionating the aqueous phase. Preferably, the aqueous phase in the first step is separated, and the obtained aqueous solution is processed by using, for example, an electrodialyzer to generate formic acid in the second step. The aqueous phase to be separated is the aqueous phase obtained after the first step has ended.

In the second step, the aqueous solution of the formate obtained in the first step may be used as it is, or may be concentrated or diluted as necessary to adjust the concentration of formate in the aqueous solution, and used. Examples of the method for diluting the aqueous solution of formate include a method in which the aqueous solution of formate is diluted by adding pure water. Examples of the method for concentrating the aqueous solution of formate include a method in which water is distilled off from the aqueous solution, and a method in which the aqueous solution is concentrated by using a separation membrane unit having a reverse osmosis membrane. In a case where the process is performed by using an electrodialyzer, loss of formate may occur due to a phenomenon of concentration diffusion in the aqueous solution of formate which has a high concentration. From the viewpoint of reducing the loss, it is preferable that the aqueous phase in the first step is separated, and the concentration of the formate is adjusted by dilution, and thereafter, the obtained aqueous solution is used in the second step. In a case where, in the first step, the aqueous solution of formate which has a high concentration is produced, and the concentration of the aqueous solution is adjusted by dilution, and then, the obtained aqueous solution is used in the second step, formic acid can be produced at a higher yield with more excellent productivity.

A degree of adjustment (preferably, dilution) of the concentration of the aqueous solution of formate which is obtained in the first step is not particularly limited. The concentration of formate in the aqueous solution after the adjustment of the concentration is preferably the concentration suitable for electrodialysis, and is preferably 2.5 mol/L or more, more preferably 3 mol/L or more and 4.75 mol/L or more, and even more preferably 5 mol/L or more. In a case where the process is performed by using an electrodialyzer, the concentration of formate is preferably 20 mol/L or less, more preferably 15 mol/L or less, and even more preferably 10 mol/L or less from the viewpoint of reducing loss of formate due to a phenomenon of concentration diffusion.

Pure water can be used for dilution. Water generated in the second step may be used for dilution. Reusing water generated in the second step for dilution is preferable since, for example, cost for waste water treatment and a load on the environment can be advantageously reduced.

In the method for producing formic acid according to the present embodiment, acid is added to the aqueous solution of formate which is obtained in the first step, and decarbonation treatment is performed, and thereafter, the resultant aqueous solution may be used in the second step. That is, the aqueous phase in the first step is separated, acid is added, and the decarbonation treatment is performed, and thereafter, the resultant aqueous solution may be used in the second step. The aqueous solution of formate which is obtained in the first step may contain unreacted carbonate or bicarbonate generated by a side reaction, and, if a solution containing carbonate or bicarbonate is subjected to electrodialysis, carbon dioxide may be generated and dialysis efficiency may be reduced. Therefore, acid is added to the aqueous solution of formate which is obtained in the first step, and decarbonation treatment is performed, and thereafter, electrodialysis is performed, whereby formic acid can be produced at a higher yield with more excellent productivity.

Examples of the acid used for the decarbonation treatment include formic acid, citric acid, acetic acid, malic acid, lactic acid, succinic acid, tartaric acid, butyric acid, fumaric acid, propionic acid, hydrochloric acid, nitric acid, and sulfuric acid. Formic acid is preferably used.

An amount of the acid to be used is preferably 50% or more and more preferably 80% or more with respect to an amount of carbonic acid existing in the solution from the viewpoint of reducing an amount of carbonic acid generated during electrodialysis treatment. Furthermore, in a case where a pH of the solution of formate is set to be approximately neutral during electrodialysis treatment, deterioration of an electrodialyzer can be inhibited. Therefore, an amount of the acid to be used is preferably 150% or less and more preferably 120% or less with respect to an amount of carbonic acid existing in the solution.

In the present embodiment, as a proportion at which formate is protonated in the second step, preferably 10% or more, more preferably 20% or more, and even more preferably 30% or more of formate is protonated with respect to an

initial molar amount of formate in the aqueous solution of formate, from the viewpoint of enhancing purity of the aqueous solution of formic acid which is to be collected.

Examples of the electrodialyzer used in the second step include a two-chamber type electrodialyzer in which a bipolar membrane, and an anion exchange membrane or a cation exchange membrane are used, and a three-chamber-type electrodialyzer in which a bipolar membrane, an anion exchange membrane, and a cation exchange membrane are used.

FIG. 1 is a schematic diagram illustrating one example of a three-chamber-type electrodialyzer. The electrodialyzer illustrated in FIG. 1 includes a plurality of bipolar membranes, a plurality of anion exchange membranes, and a plurality of cation exchange membranes. The bipolar membranes, the anion exchange membranes, and the cation exchange membranes are disposed between an anode and a cathode, so that a base tank, a sample tank (salt tank), and an acid tank are formed. The aqueous solution of formate is circulated and supplied to the sample tank while electricity is caused to pass through the electrodialyzer, so that formate can be converted to formic acid, formic acid can be collected from the acid tank, water can be collected from the sample tank, and hydroxide can be collected from the base tank.

The two-chamber-type electrodialyzer includes, for example, a plurality of bipolar membranes and a plurality of cation exchange membranes. The bipolar membranes and the cation exchange membranes alternate between an anode and a cathode, so that a salt chamber is formed between each bipolar membrane and the cation exchange membrane disposed on the cathode side of the bipolar membrane, and a base tank is formed between each bipolar membrane and the cation exchange membrane disposed on the anode side of the bipolar membrane. The aqueous solution of formate is circulated and supplied to the salt chamber while electricity is caused to pass through the electrodialyzer, so that hydroxide is generated in the base tank, and formate that is circulated and supplied to the salt chamber is converted to formic acid.

In the second step, by using the electrodialyzer, formate can be protonated by a simple method to obtain a solution of formic acid.

[System for producing formic acid]

As shown in FIG. 2, a production system 100 for producing formic acid according to the present embodiment includes, for example, a production device 10 for producing formate, and an electrodialyzer 30. The production system 100 may further include a diluting device 20 and a storage portion 40 for storing dilution water, and may further include a carbon dioxide cylinder 60 for introducing carbon dioxide into the production device 10, and a hydrogen cylinder 50 for introducing hydrogen into the production device 10. The concentration and the pressure of each of carbon dioxide and hydrogen can be adjusted by a valve 1 and a valve 2 disposed in piping L1 and piping L2, respectively.

Formate produced by the production device 10 is supplied to the electrodialyzer 30 as the aqueous solution of formate by separating the aqueous phase. At this time, as shown in FIG. 2, the concentration of formate in the aqueous solution may be adjusted by sending the aqueous solution of formate to the diluting device 20 beforehand through a flow path L3, and diluting the aqueous solution of formate by the diluting device 20.

In the aqueous solution in which the concentration of formate has been adjusted by the diluting device 20, at least a part of formate is protonated by the electrodialyzer 30. Thus, formic acid and water are generated from formate. The generated formic acid can be taken out through a flow path L5. The generated water may be sent to the storage portion 40 through a flow path L7.

A part of formic acid generated by the electrodialyzer 30 may be sent to the storage portion 40 through a flow path L6. The storage portion 40 may further include a water supply portion 70 and a formic acid supply portion 80. By supplying the aqueous solution of formic acid which has been prepared by the storage portion 40 to the diluting device 20 through a flow path L9, the aqueous solution of formate may be subjected to decarbonation treatment. Each of flow paths of the production system 100 may include a valve for adjusting pressure and a supply amount.

The production system 100 of the present embodiment can produce formic acid at a high yield with excellent productivity.

[Catalyst composition]

A catalyst composition according to a third embodiment of the present invention includes a catalyst and an antioxidant and satisfies at least one selected from the group consisting of (A) to (D) below.

(A) A phase-transfer catalyst is further included.
(B) The catalyst is a compound in which, in General formula (1A), Y is an atomic group having a phosphorus atom and a substituent and the substituent is an alkyl group.
(C) The content of the antioxidant is 10 equivalents or more with respect to 1 equivalent of the catalyst.
(D) The antioxidant includes a phosphorus-based antioxidant.

[Chem. 18]

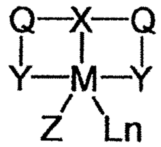

(1A)

(In General formula (1A),

X represents an atomic group including typical elements of Groups 13 to 15 that can coordinate to M,
each Q independently represents a cross-linked structure including typical elements of Groups 14 to 16 and linking Y and X,
each Y independently represents an atomic group including typical elements of Groups 14 to 16 that can coordinate to M, and M represents a metal atom,
Z represents an anionic ligand,
n represents 0 to 3, and
in a case where the number of Ls is plural, each L independently represents a neutral or anionic ligand.)

Since the catalyst composition contains the antioxidant, a decrease in catalytic efficiency in the catalytic reaction can be suppressed.

The catalyst composition of the present embodiment may satisfy (A) above. That is, the present invention provides a catalyst composition including a catalyst, an antioxidant, and a phase-transfer catalyst. The phase-transfer catalyst is, for example, the compound described above as a phase-transfer catalyst used in the catalytic reaction method according to the first embodiment, and the preferable ranges are also the same as in the first embodiment. Such a catalyst composition is useful, for example, for a reaction in a two-phase system, and can further suppress a decrease in catalytic efficiency in the catalytic reaction.

The catalyst is, for example, the compound described above as a catalyst used in the catalytic reaction method according to the first embodiment, and the preferable ranges are also the same as in the first embodiment.

The catalyst composition of the present embodiment may satisfy (B) above. That is, the present invention, in another aspect thereof, provides a catalyst composition including a catalyst and an antioxidant, wherein the catalyst is a compound in which, in General formula (1A), Y is an atomic group having a phosphorus atom and a substituent and the substituent is an alkyl group. The catalyst may be a compound in which, in General formulae (2A) and (3A) described above in the first embodiment, $Y_1$ is a phosphorus atom and R is an alkyl group, or may be a compound in which $R_1$ is an alkyl group in General formula (4A).

The catalyst composition of the present embodiment may satisfy (C) above. That is, the present invention, in another aspect thereof, provides a catalyst composition including a catalyst and an antioxidant, wherein the content of the antioxidant is 10 equivalents or more with respect to 1 equivalent of the catalyst. The content of the antioxidant is, for example, 10 equivalents or more and 10000 equivalents or less, preferably 100 equivalents or more and 10000 equivalents or less, and more preferably 100 equivalents or more and 1000 equivalents or less with respect to 1 equivalent of the catalyst.

The antioxidant is, for example, the antioxidant described above as an antioxidant used in the catalytic reaction method according to the first embodiment. It is preferable that the antioxidant includes at least one selected from the group consisting of a phosphorus-based antioxidant, an amine-based antioxidant, and a phenolic-based antioxidant, and it is more preferable that the antioxidant includes a phosphorus-based antioxidant.

The catalyst composition of the present embodiment may satisfy at least two selected from the group consisting of (A) to (D) above. The catalyst composition of the present embodiment satisfies, for example, (A) and at least one selected from the group consisting of (B) to (D).

The catalyst composition of the present embodiment is, for example, a catalyst composition for generating an organic compound, and is preferably a catalyst composition for generating a formate. In addition, the catalyst composition of the present embodiment is, for example, a catalyst composition for a hydrogenation reaction. The starting compound is, for example, at least one selected from the group consisting of carbon dioxide, bicarbonate, and carbonate.

The starting compound may be brought into contact with the catalyst composition of the present embodiment to generate an organic compound. The contact between the catalyst composition and the starting compound is performed, for example, by mixing the catalyst composition in a solution containing the starting compound, mixing the catalyst composition and the starting compound in a solvent, or mixing a solution containing the starting compound and a solution containing the catalyst composition.

The catalyst composition of the present embodiment is used, for example, in the catalytic reaction method according to the first embodiment of the present invention or the method for producing an organic compound according to the second embodiment.

The catalyst composition of the present embodiment may further include a solvent. The solvent may be at least one selected from the group consisting of an organic solvent and an aqueous solvent. In a preferred embodiment of the present invention, the catalyst composition includes only an organic solvent as a solvent. In another preferred embodiment of the present invention, the catalyst composition includes an organic solvent and an aqueous solvent. As the organic solvent, for example, the organic solvent described above in the first embodiment is used. The organic solvent is preferably toluene or dioxane, and more preferably toluene. As the aqueous solvent, for example, the aqueous solvent described above in the first embodiment is used. Examples

The present invention will be more specifically described below by way of examples and comparative examples. However, the present invention is not limited to the examples. In each example, a formate was synthesized by a hydrogenation reaction of the above-described starting compound with hydrogen using a catalyst, and the effect of suppressing a decrease in catalytic efficiency was evaluated.

[Synthesis of catalyst]

(Catalyst 1)

A catalyst 1 was synthesized by the following operation. 40 mg (0.1 mmol) of the following ligand A was added to a THF (tetrahydrofuran) (5 ml) suspension of 95.3 mg (0.1 mmol) of [$RuHCl(PPh_3)_3(CO)$] in an inert atmosphere, and the mixture was stirred and heated at 65°C for three hours, to cause a reaction. Thereafter, the resultant product was cooled to room temperature (25°C). The obtained yellow solution was filtered, and the filtrate was evaporated to dryness under a vacuum. The obtained yellow residual oil was dissolved in a small amount of THF (1 mL), and hexane (10 mL) was slowly added to precipitate a yellow solid product, and the solid product was filtered. The filtrate was dried under a vacuum, and the following catalyst 1 (55 mg, yield of 97%) was obtained as yellow crystals. In the following catalyst 1 and ligand A, tBu represents a tertiary butyl group.

[Chem. 19]

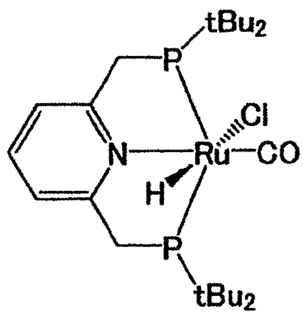

1

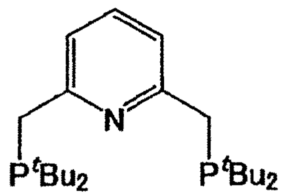

A

$^{31}P\{^1H\}(C_6D_6)$: 90.8(s), $^1H(C_6D_6)$: -14.54 (t, 1H, J=20.0 Hz), 1.11 (t, 18H, J=8.0 Hz), 1.51 (t, 18H, J=8.0 Hz), 2.88 (dt, 2H, J=16.0 Hz, J=4.0 Hz), 3.76 (dt, 2H, J=16.0 Hz, J=4.0 Hz), 6.45 (d, 2H, J=8.0 Hz), 6.79 (t, 1H, J=8.0 Hz). $^{13}C\{^1H\}$ NMR($C_6D_6$): 29.8(s), 30.7(s), 35.2 (t, J=9.5 Hz), 37.7 (t, J=6.0 Hz), 37.9 (t, J=6.5 Hz), 119.5 (t, J=4.5 Hz), 136.4(s), 163.4 (t, J=5.0 Hz), 209.8(s).

[Calculation of TON and yield]

In the following examples and comparative examples, the TON of a catalyst and the yield of a formate (potassium formate) were calculated by the following method.

First, the amount of substance of the formate included in the aqueous phase was determined as follows. 100 μL was taken from the aqueous phase obtained after the reaction, and 300 μL of dimethyl sulfoxide was added as a reference substance and dissolved in 500 μL of Deuterium oxide. Thus, a measurement sample was prepared. $^1H$ NMR measurement was performed on this measurement sample. From the obtained NMR spectrum, an integral value Ia of the peak derived from potassium formate and an integral value Ib of the peak derived from dimethyl sulfoxide were identified. An amount of substance X (mol) of potassium formate was calculated by the following formula (1).

$$X = (W/M) \times \{Ia/(Ib/R)\} \times (A/B) \quad (1)$$

(In formula (1), W is the weight (g) of dimethyl sulfoxide used for the determination of potassium formate,
M is the molecular weight of dimethyl sulfoxide,
R is the ratio of the number of protons of dimethyl sulfoxide per molecule to the number of protons of potassium formate per molecule,
Ia is the integral value of the NMR peak derived from potassium formate,
Ib is the integral value of the NMR peak derived from dimethyl sulfoxide,
A is the mass (g) of the aqueous phase obtained after the reaction, and
B is the mass (g) of the aqueous solution used for the determination of potassium formate.)

Then, the TON of the catalyst was calculated by the following formula (2) based on the amount of substance X (mol) of the generated formate and an amount of substance Y (mol) of the catalyst used in the reaction.

$$\text{TON of catalyst} = X/Y \quad (2)$$

Furthermore, the yield (%) of the formate was calculated by the following formula (3) based on the amount of substance X (mol) of the generated formate, and the sum Z (mol) of amounts of substance of carbon dioxide, bicarbonate, and carbonate used in the reaction (amount of substance of potassium bicarbonate in the following examples and comparative examples).

$$\text{Yield of formate} = 100 \times X/Z \quad (3)$$

[Example 1]

In a nitrogen gas atmosphere, 50 mL of water, 0.25 mol of potassium bicarbonate, 50 mL of toluene, 0.75 μmol of the catalyst 1, 2.7 mmol of methyltrioctylammonium chloride, and 0.3 mmol of tris(2,4-di-tert-butylphenyl)phosphite as an antioxidant were put in a reactor equipped with a U-shaped paddle stirring blade. Then, as the first reaction, hydrogen gas was injected until reaching 5 MPa, then the temperature was increased to 90°C, and the mixture was stirred at 800 rpm for 16 hours. After stirring, the mixture was cooled to room temperature, and the pressure was carefully released after cooling. The inside of the reactor was replaced with nitrogen gas.

In a nitrogen gas atmosphere, the reaction solution was taken out from the reactor, and the organic phase and the aqueous phase were separated from each other. Using this aqueous phase, the TON of the catalyst and the yield of the formate in the first reaction were calculated by the above-described method.

In a nitrogen gas atmosphere, toluene was added to the separated organic phase to increase the volume to 50 mL. In a nitrogen gas atmosphere, the total amount (50 mL) of the organic phase, 50 mL of water, and 0.25 mol of potassium bicarbonate were put in a reactor equipped with a U-shaped paddle stirring blade. Then, as the second reaction, hydrogen gas was injected until reaching 5 MPa, then the temperature was increased to 90°C, and the mixture was stirred at 800 rpm for 16 hours. After stirring, the mixture was cooled to room temperature, and the pressure was carefully released after cooling. The inside of the reactor was replaced with nitrogen gas.

In a nitrogen gas atmosphere, the reaction solution was taken out from the reactor, and the organic phase and the aqueous phase were separated from each other. As in the first reaction, the TON of the catalyst and the yield of the formate in the second reaction were calculated by the above-described method.

In a nitrogen gas atmosphere, toluene was added to the separated organic phase to increase the volume to 50 mL. In a nitrogen gas atmosphere, the total amount (50 mL) of the organic phase, 50 mL of water, and 0.25 mol of potassium bicarbonate were put in a reactor equipped with a U-shaped paddle stirring blade. Then, as the third reaction, hydrogen gas was injected until reaching 5 MPa, then the temperature was increased to 90°C, and the mixture was stirred at 800 rpm for 16 hours. After stirring, the mixture was cooled to room temperature, and the pressure was carefully released after cooling. The inside of the reactor was replaced with nitrogen gas.

In a nitrogen gas atmosphere, the reaction solution was taken out from the reactor, and the organic phase and the aqueous phase were separated from each other. As in the first and second reactions, the TON of the catalyst and the yield of the formate in the third reaction were calculated by the above-described method.

[Example 2]

A reaction was performed in the same manner as in Example 1, except that the antioxidant was changed to 3,9-bis(2,4-di-tert-butylphenoxy)-2,4,8,10-tetraoxa-3,9-diphosphaspiro[5.5]undecane. As in Example 1, the TON of the catalyst and the yield of the formate in each of the first to third reactions were calculated.

[Example 3]

A reaction was performed in the same manner as in Example 1, except that the antioxidant was changed to 2,6-di-tert-butyl-p-cresol. As in Example, the TON of the catalyst and the yield of the formate in each of the first to third reactions were calculated.

[Example 4]

A reaction was performed in the same manner as in Example 1, except that the antioxidant was changed to 6-ethoxy-2,2,4-trimethyl-1,2-dihydroquinoline. As in Example 1, the TON of the catalyst and the yield of the formate in each of the first to third reactions were calculated.

[Comparative Example 1]

A reaction was performed in the same manner as in Example 1, except that no antioxidant was added. As in Example 1, the TON of the catalyst and the yield of the formate in each of the first to third reactions were calculated.

Table 1 shows the TON of the catalyst and the yield in each reaction in Examples 1 to 4 and Comparative Example 1. The total-TON shown in Table 1 means the ratio of the sum of the amounts of substance of the formate generated in the first reaction to the third reaction to the amount of substance of the used catalyst.

[Table 1]

| | Antioxidant | Antioxidant category | TON First time | TON Second time | TON Third time |
|---|---|---|---|---|---|
| Example 1 | Tris(2,4-di-tert-butylphenyl) phosphite | Phosphorus-based | $2.54\times10^5$ | $2.60\times10^5$ | $2.59\times10^5$ |
| Example 2 | 3,9-bis(2,4-di-tert-butylphenoxy)-2,4,8,10-tetraoxa-3,9-diphosphaspiro[5.5]undecane | Phosphorus-based | $2.43\times10^5$ | $2.61\times10^5$ | $2.63\times10^5$ |
| Example 3 | 2,6-di-tert-butyl-p-cresol | Phenolic-based | $2.54\times10^5$ | $2.25\times10^5$ | $1.21\times10^5$ |
| Example 4 | 6-ethoxy-2,2,4-trimethyl-1,2-dihydroquinoline | Amine-based | $2.58\times10^5$ | $2.63\times10^5$ | $2.25\times10^5$ |
| Comparative Example 1 | None | - | $2.38\times10^5$ | $1.51\times10^5$ | $7.76\times10^4$ |

[Table 1] (cont.)

| | Yield First time (%) | Yield Second time (%) | Yield Third time (%) | Total-TON |
|---|---|---|---|---|
| Example 1 | 76 | 79 | 78 | $7.73\times10^5$ |
| Example 2 | 73 | 78 | 79 | $7.67\times10^5$ |
| Example 3 | 76 | 67 | 36 | $5.99\times10^5$ |
| Example 4 | 77 | 79 | 67 | $7.46\times10^5$ |
| Comparative Example 1 | 72 | 45 | 23 | $4.67\times10^5$ |

As indicated in Table 1, in Examples 1 to 4 in which the reaction was performed in a two-phase system including the antioxidant, the TON was maintained even when the reaction was performed repeatedly two or more times, as compared to Comparative Example 1 in which no antioxidant was included. Therefore, a decrease in catalytic efficiency was suppressed in Examples 1 to 4. From Table 1, it can be seen that the use of phosphorus-based antioxidants is particularly suitable for suppressing a decrease in catalytic efficiency. In addition, even when the antioxidant was added, the yield was a sufficient value for practical use.

[Example 5]

A reaction was performed in the same manner as in Example 1, except that the amount of the catalyst to be used was changed to 0.175 μmol. As in Example 1, the TON of the catalyst and the yield of the formate in each of the first to third reactions were calculated.

[Example 6]

A reaction was performed once in the same manner as in Example 5, except that the antioxidant was changed to triphenyl phosphite, and the TON of the catalyst and the yield of the formate in the first reaction were calculated.

[Example 7]

A reaction was performed in the same manner as in Example 6, except that the antioxidant was changed to tetraalkyl(C12-15)-4,4'-isopropylidene diphenyl diphosphate (ADK STAB 1500), and the TON of the catalyst and the yield of the formate in the first reaction were calculated.

[Example 8]

A reaction was performed in the same manner as in Example 6, except that the antioxidant was changed to triisodecyl phosphite, and the TON of the catalyst and the yield of the formate in the first reaction were calculated.

[Example 9]

A reaction was performed in the same manner as in Example 6, except that the antioxidant was changed to 3,9-bis(2,4-di-tert-butylphenoxy)-2,4,8,10-tetraoxa-3,9-diphosphaspiro[5.5]undecane, and the TON of the catalyst and the yield of the formate in the first reaction were calculated.

[Comparative Example 2]

A reaction was performed in the same manner as in Example 6, except that no antioxidant was added, and the TON of the catalyst and the yield of the formate in the first reaction were calculated.

Table 2 shows the TON of the catalyst and the yield in each reaction in Examples 5 to 9 and Comparative Example 2.

[Table 2]

| | Antioxidant | Antioxidant category | TON First time | TON Second time | TON Third time |
|---|---|---|---|---|---|
| Example 5 | Tris(2,4-di-tert-butylphenyl) phosphite | Phosphorus-based | $1.06\times10^6$ | $1.02\times10^6$ | $8.45\times10^5$ |
| Example 6 | Triphenyl phosphite | Phosphorus-based | $1.06\times10^6$ | - | - |
| Example 7 | Tetraalkyl(C12-15)-4,4'-isopropylidene diphenyl diphosphite | Phosphorus-based | $9.50\times10^5$ | - | - |
| Example 8 | Triisodecyl phosphite | Phosphorus-based | $9.34\times10^5$ | - | - |
| Example 9 | 3,9-bis(2,4-di-tert-butylphenoxy)-2,4,8,10-tetraoxa-3,9-diphosphaspiro[5.5]undecane | Phosphorus-based | $9.51\times10^5$ | - | - |
| Comparative Example 2 | None | - | $2.65\times10^4$ | - | - |

[Table 2] (cont.)

| | Yield First time (%) | Yield Second time (%) | Yield Third time (%) | Total-TON |
|---|---|---|---|---|
| Example 5 | 74 | 72 | 59 | $2.93\times10^6$ |
| Example 6 | 74 | - | - | - |
| Example 7 | 66 | - | - | - |
| Example 8 | 65 | - | - | - |
| Example 9 | 67 | - | - | - |
| Com parative Example 2 | 19 | - | - | - |

As indicated in Table 2, Examples 5 to 9 exhibited higher TONs and yields than Comparative Example 2 in which no antioxidant was included. Furthermore, Examples 5 to 9 exhibited higher TONs than Examples 1 to 4, and in particular, Examples 5 and 6 exhibited high TONs. In addition, in Example 5, the TON was almost maintained even when the reaction was performed repeatedly two or more times. Therefore, in Examples 5 to 9, the TON of the catalyst was further improved.

[Synthesis of catalysts]

(Catalyst 2)

A catalyst 2 was synthesized by the following operation. 138 mg (0.3 mmol, 1.0 equivalent) of bromopentacarbonyl manganese was put in a 50 mL Schlenk tube. Then, 10 mL of degassed toluene was added in an argon atmosphere. In addition, 300 mg (0.66 mmol, 1.3 equivalents) of the following ligand B was added. After refluxing at 115°C for 18 hours, a yellow precipitate was filtered off. The filtrate was washed three times with hexane (10 mL) and extracted with dichloromethane. The solvent was evaporated from the extract to obtain the following catalyst 2 (300 mg, yield: 92%) as a yellow powder. In the catalyst 2 and the ligand B shown below, Ph represents a phenyl group.

[Chem. 20]

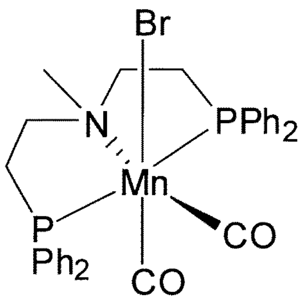

2

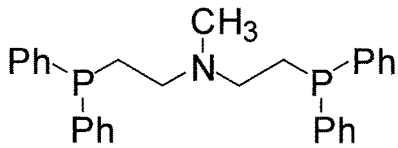

B

(Catalyst 3)

A catalyst 3 was synthesized by the following operation. Bis(2-diethylphosphinoethyl)amine (1 mmol) was added to a suspension of bromopentacarbonyl manganese (1 mmol) in toluene (5 mL), and the suspension was stirred at 100°C for 24 hours in an argon atmosphere to perform a reaction. Then, the reaction mixture was cooled to room temperature and evaporated to dryness. The obtained yellow solid was washed several times with toluene. The washed matter was dried under vacuum to obtain the following catalyst 3 as a light yellow solid. In the catalyst 3 shown below, Et represents an ethyl group.

[Chem. 21]

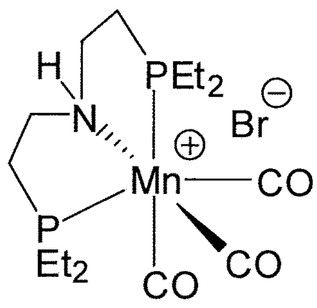

**3**

[Example 10]

In an argon gas atmosphere, 1 mL of water, 2.0 mmol potassium bicarbonate, 1 mL of toluene, 5.3 μmol of the catalyst 2, 54 μmol of methyltrioctylammonium chloride, and 30 μmol of tris(2,4-di-tert-butylphenyl)phosphite as an antioxidant were put in a reactor equipped with a stirrer bar. Then, hydrogen gas was injected up to 5.5 MPa, then the temperature was increased to 90°C, and the mixture was stirred at 800 rpm for 20 hours. After stirring, the mixture was cooled to room temperature, and the pressure was carefully released after cooling. The reaction solution was taken out from the reactor, and the organic phase and the aqueous phase were separated from each other. Using this aqueous phase, the TON of the catalyst and the yield of the formate were calculated by the above-described method.

[Comparative Example 3]

A reaction was performed in the same manner as in Example 10, except that no antioxidant was added, and the TON of the catalyst and the yield of the formate in the first reaction were calculated.

[Example 11]

A reaction was performed in the same manner as in Example 10, except that the amount of the catalyst to be used was changed to 12.6 μmol and toluene was changed to 1,4-dioxane, and the TON of the catalyst and the yield of the formate were calculated.

[Comparative Example 4]

A reaction was performed in the same manner as in Example 11, except that no antioxidant was added, and the TON of the catalyst and the yield of the formate in the first reaction were calculated.

[Example 12]

A reaction was performed in the same manner as in Example 11, except that the catalyst to be used was changed to 9.6 μmol of the catalyst 3, and the TON of the catalyst and the yield of the formate were calculated.

[Comparative Example 5]

A reaction was performed in the same manner as in Example 12, except that no antioxidant was added, and the TON of the catalyst and the yield of the formate in the first reaction were calculated.

Table 3 shows the TON of the catalyst and the yield in each reaction in Examples 10 to 12 and Comparative Examples 3 to 5.

[Table 3]

| | Antioxidant | TON | Yield (%) |
|---|---|---|---|
| Example 10 | Tris(2,4-di-tert-butylphenyl)phosphite | 174 | 19 |

(continued)

| | Antioxidant | TON | Yield (%) |
|---|---|---|---|
| Comparative Example 3 | None | 123 | 13 |
| Example 11 | Tris(2,4-di-tert-butylphenyl)phosphite | 321 | 81 |
| Comparative Example 4 | None | 282 | 71 |
| Example 12 | Tris(2,4-di-tert-butylphenyl)phosphite | 365 | 70 |
| Comparative Example 5 | None | 267 | 51 |

As indicated in Table 3, even when the manganese catalyst was used, the hydrogenation reaction of the starting compound with hydrogen progressed, and the formate was synthesized. As indicated in Table 3, Example 10 exhibited a higher TON and yield than Comparative Example 3 in which no antioxidant was included. Example 11 exhibited a higher TON and yield than Comparative Example 4 in which no antioxidant was included. Example 12 exhibited a higher TON and yield than Comparative Example 5 in which no antioxidant was included.

[Protonation of formate]

In the following Reference examples 1 and 2, formate was protonated by using an electrodialyzer (ACILYZER EX3B manufactured by ASTOM Corporation).

[Reference example 1]

In a base tank of the electrodialyzer, 165 g of potassium hydroxide which was dissolved in 500 mL of water was put. In a salt tank, 500 mL of a 5 mol/L potassium formate aqueous solution was put. In an acid tank, 500 mL of a 4.35 mol/L formic acid aqueous solution was put. Electrodialysis was performed at a voltage of 28 V for 80 minutes. After the dialysis ended, 100 μL of the solution (acid liquid) in the acid tank was taken and dissolved in 500 μL of Deuterium oxide, and 300 μL of dimethyl sulfoxide was additionally added as an internal standard, and $^1H$ NMR measurement was performed, to quantify formic acid in the acid liquid having been subjected to the dialysis.

[Reference example 2]

In a base tank of the electrodialyzer, 165 g of potassium hydroxide which was dissolved in 500 mL of water was put. In a salt tank, 500 mL of an aqueous solution containing 4.75 mol/L of potassium formate and 0.25 mol/L of potassium bicarbonate was put. In an acid tank, 500 mL of a 4.81 mol/L formic acid aqueous solution was put. Electrodialysis was performed at a voltage of 28 V for 80 minutes. After the dialysis ended, 100 μL of the solution (acid liquid) in the acid tank was taken and dissolved in 500 μL of Deuterium oxide, and 300 μL of dimethyl sulfoxide was additionally added as an internal standard, and $^1H$ NMR measurement was performed, to quantify formic acid in the acid liquid having been subjected to the dialysis.

Indication in Table 4 is for Reference examples 1 and 2. In Table 4, an initial formate proportion represents a percentage of a substance amount X2 of formate relative to the total of the substance amount (substance amount X2) of formate and a substance amount of bicarbonate in the salt tank prior to the electrodialysis. An initial formate concentration represents a molar concentration of formate in the salt tank prior to the electrodialysis. An initial bicarbonate concentration represents a molar concentration of bicarbonate in the salt tank prior to the electrodialysis. An initial formic acid concentration represents a molar concentration of formic acid in the acid tank prior to the electrodialysis. A final formic acid concentration represents a molar concentration of formic acid in the acid tank after the end of the electrodialysis. A yield of formic acid represents a percentage of a substance amount (mol) of formic acid obtained through the electrodialysis, relative to the substance amount (substance amount X2) of formate used for the electrodialysis.

[Table 4]

| | | Reference example 1 | Reference example 2 |
|---|---|---|---|
| Prior to dialysis | Initial formate proportion (%) | 100 | 95 |
| | Initial formate concentration (mol/L) | 5 | 4.75 |
| | Initial bicarbonate concentration (mol/L) | 0 | 0.25 |
| | Initial formic acid concentration (mol/L) | 4.35 | 4.81 |

(continued)

| | | Reference example 1 | Reference example 2 |
|---|---|---|---|
| After dialysis | Final formic acid concentration (mol/L) | 6.28 | 6.68 |
| | Yield of formic acid (%) | 50.3 | 51.8 |

As indicated in Table 4, formic acid was able to be obtained from formate through the electrodialysis. In Reference example 2 in which bicarbonate was contained in the salt tank, formic acid was able to be obtained at a yield equivalent to that in Reference example 1 in which no bicarbonate was contained in the salt tank.

INDUSTRIAL APPLICABILITY

With the catalytic reaction method and the method for producing an organic compound according to the present embodiments, for example, it is possible to efficiently produce the target organic compound at low cost.

## Claims

1. A catalytic reaction method comprising causing a starting compound to react in the presence of a solvent using a catalyst, wherein

   the solvent includes an organic solvent, an aqueous solvent, and an antioxidant, and
   the reaction is performed in a two-phase system in which the organic solvent and the aqueous solvent are separate.

2. The catalytic reaction method according to claim 1, wherein an organic phase including the organic solvent includes the catalyst, and an aqueous phase including the aqueous solvent includes the starting compound.

3. The catalytic reaction method according to claim 1, wherein an organic phase including the organic solvent includes the antioxidant.

4. The catalytic reaction method according to claim 1, wherein the antioxidant includes at least one selected from the group consisting of a phosphorus-based antioxidant, an amine-based antioxidant, and a phenolic-based antioxidant.

5. The catalytic reaction method according to claim 4, wherein the antioxidant includes a phosphorus-based antioxidant.

6. The catalytic reaction method according to claim 5, wherein the phosphorus-based antioxidant is a compound represented by the following Chemical formula (1B),

[Chem. 1]

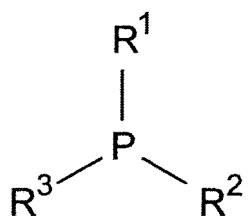

(1B)

(in Chemical formula (1B), $R^1$, $R^2$, and $R^3$ each independently represent a hydrogen atom or an optional substituent).

7. The catalytic reaction method according to claim 5, wherein the phosphorus-based antioxidant is a compound represented by the following Chemical formula (1C),

[Chem. 2]

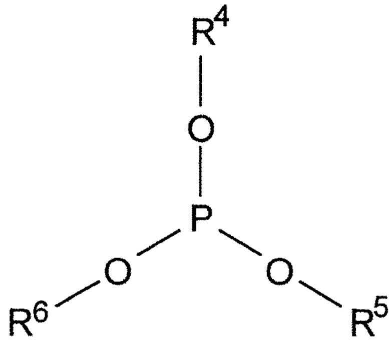

(1C)

(in Chemical formula (1C), $R^4$, $R^5$, and $R^6$ each independently represent an optional substituent).

**8.** The catalytic reaction method according to claim 7, wherein, in the Chemical formula (1C), $R^4$, $R^5$, and $R^6$ are each independently the following Chemical formula (1D),

[Chem. 3]

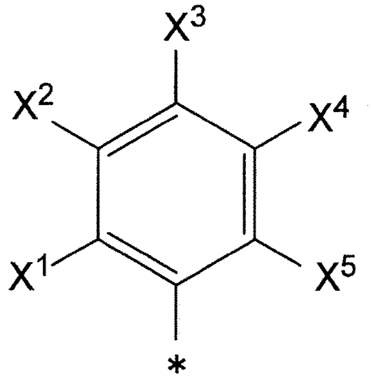

(1D)

(in Chemical formula (1D), * represents a bond, and $X^1$, $X^2$, $X^3$, $X^4$, and $X^5$ each independently represent a hydrogen atom or a hydrocarbon group).

**9.** The catalytic reaction method according to claim 1, wherein the reaction is a hydrogenation reaction of the starting compound with hydrogen, and a hydrogenated compound of the starting compound is obtained by the reaction.

**10.** The catalytic reaction method according to claim 9, wherein the starting compound is at least one selected from the group consisting of carbon dioxide, bicarbonate, and carbonate, and a formate is obtained from the starting compound by the reaction.

**11.** The catalytic reaction method according to claim 1, wherein the catalyst is at least one selected from the group consisting of a metal complex represented by the following General formula (1A), a tautomer of the metal complex, a stereoisomer of the metal complex, and salts thereof,

[Chem. 4]

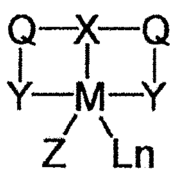

(1A)

(in General formula (1A),

X represents an atomic group including typical elements of Groups 13 to 15 that can coordinate to M,
each Q independently represents a cross-linked structure including typical elements of Groups 14 to 16 and linking Y and X,
each Y independently represents an atomic group including typical elements of Groups 14 to 16 that can coordinate to M, and M represents a metal atom,
Z represents an anionic ligand,
n represents 0 to 3, and
in a case where the number of Ls is plural, each L independently represents a neutral or anionic ligand).

**12.** The catalytic reaction method according to claim 11, wherein the metal complex represented by the General formula (1A) is a metal complex represented by the following General formula (2A),

[Chem. 5]

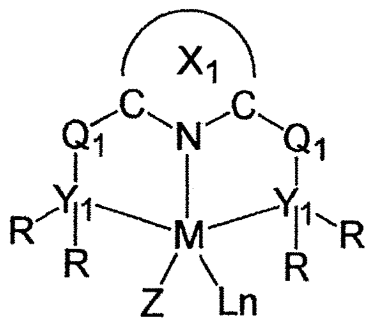

(2A)

(in General formula (2A),

$X_1$ represents a heteroaromatic ring formed with two carbon atoms and a nitrogen atom, and may have a substituent, or may combine with another substituent to form a ring,
each $Q_1$ independently represents $CH_2$, NH, or O, and $CH_2$ and NH may each further have a substituent,
each $Y_1$ independently represents a phosphorus atom or a nitrogen atom,
each R independently represents an alkyl group, an aryl group, or an aralkyl group, and may further have a substituent,
M represents a metal atom,
Z represents an anionic ligand,
n represents 0 to 3, and
in a case where the number of Ls is plural, each L independently represents a neutral or anionic ligand).

**13.** The catalytic reaction method according to claim 12, wherein the metal complex represented by the General formula (2A) is a metal complex represented by the following General formula (3A),

[Chem. 6]

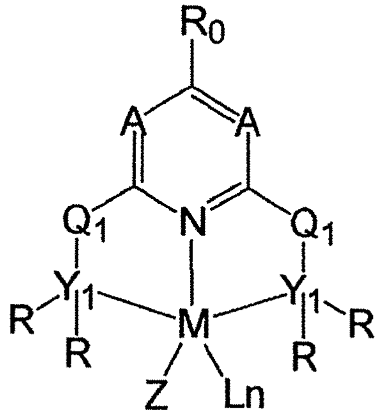

(3A)

(in General formula (3A),

$R_0$ represents a hydrogen atom or an alkyl group,
each A independently represents CH, $CR_5$, or N, and $R_5$ represents an alkyl group, an aryl group, an aralkyl group, an amino group, a hydroxy group, or an alkoxy group,
each $Q_1$ independently represents $CH_2$, NH, or O, and $CH_2$ and NH may each further have a substituent,
each $Y_1$ represents a phosphorus atom or a nitrogen atom,
each R independently represents an alkyl group, an aryl group, or an aralkyl group, and may further have a substituent,
M represents a metal atom,
Z represents an anionic ligand,
n represents 0 to 3, and
in a case where the number of Ls is plural, each L independently represents a neutral or anionic ligand).

**14.** The catalytic reaction method according to claim 11, wherein the metal atom represented by M is ruthenium.

**15.** The catalytic reaction method according to claim 1, wherein the organic solvent includes toluene.

**16.** A method for producing an organic compound, comprising synthesizing an organic compound from the starting compound by the catalytic reaction method according to any one of claims 1 to 15.

**17.** A catalyst composition comprising:

a catalyst;
an antioxidant; and
a phase-transfer catalyst.

**18.** The catalyst composition according to claim 17, wherein the antioxidant includes at least one selected from the group consisting of a phosphorus-based antioxidant, an amine-based antioxidant, and a phenolic-based antioxidant.

**19.** The catalyst composition according to claim 18, wherein the antioxidant includes a phosphorus-based antioxidant.

**20.** The catalyst composition according to claim 17, wherein the catalyst is at least one selected from the group consisting of a metal complex represented by the following General formula (1A), a tautomer of the metal complex, a stereoisomer of the metal complex, and salts thereof,

[Chem. 7]

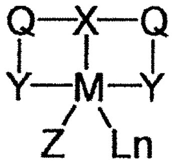

(1A)

(in General formula (1A),

X represents an atomic group including typical elements of Groups 13 to 15 that can coordinate to M,
each Q independently represents a cross-linked structure including typical elements of Groups 14 to 16 and linking Y and X,
each Y independently represents an atomic group including typical elements of Groups 14 to 16 that can coordinate to M, and M represents a metal atom,
Z represents an anionic ligand,
n represents 0 to 3, and
in a case where the number of Ls is plural, each L independently represents a neutral or anionic ligand).

**21.** The catalyst composition according to claim 17, being for generating a formate.

**22.** The catalyst composition according to claim 17, being for a hydrogenation reaction.

**23.** The catalyst composition according to claim 17, further comprising a solvent.

**24.** The catalyst composition according to claim 23, wherein the solvent is at least one selected from the group consisting of an organic solvent and an aqueous solvent.

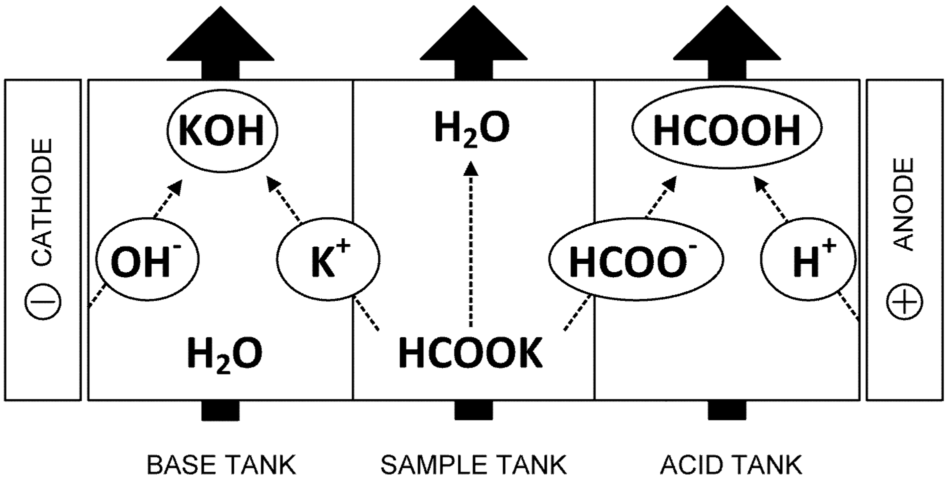
KOH
$H_2O$
HCOOH
CATHODE
ANODE
$OH^-$
$K^+$
$HCOO^-$
$H^+$
$H_2O$
HCOOK
BASE TANK
SAMPLE TANK
ACID TANK

FIG.1

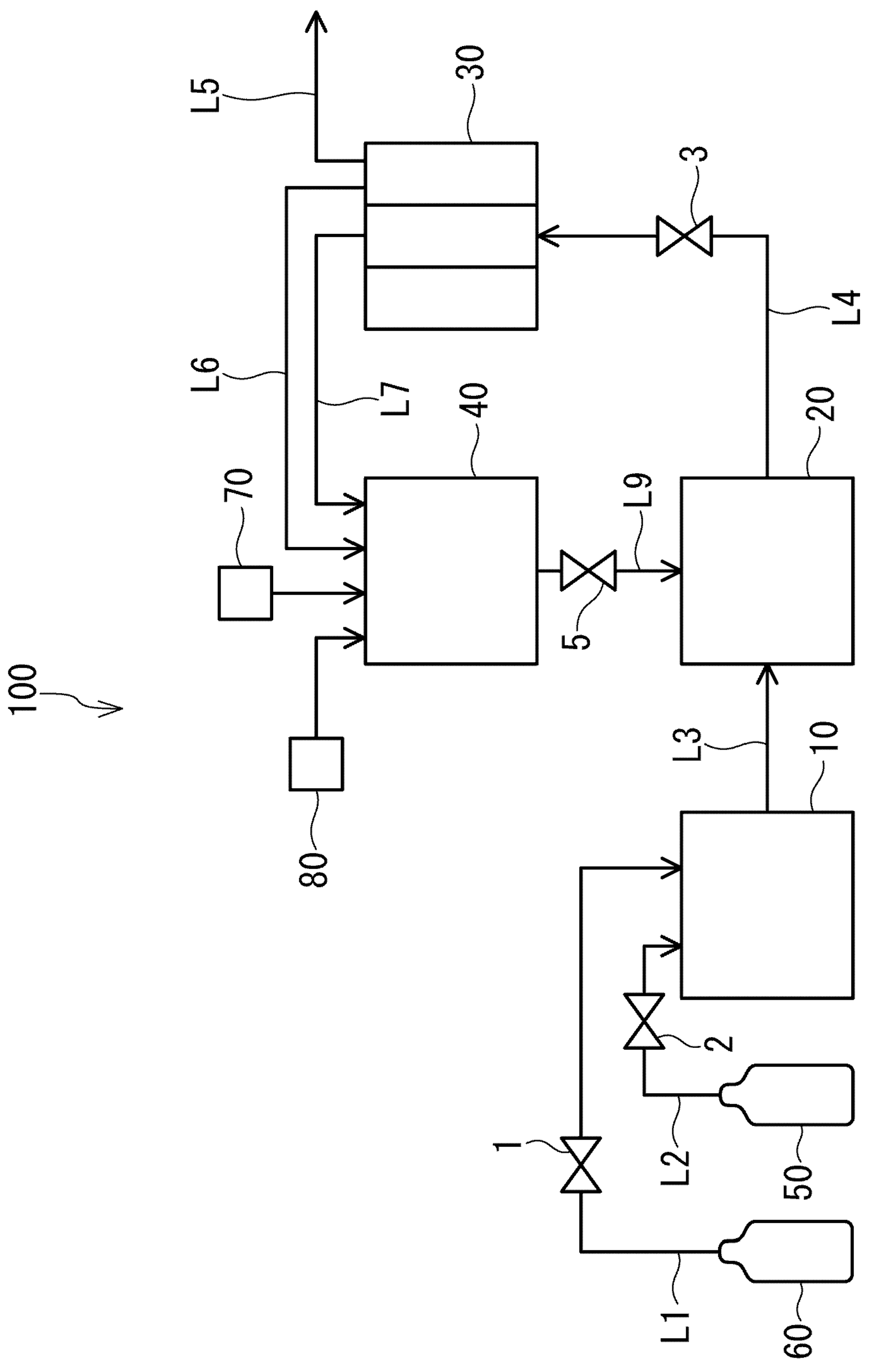


FIG.2

**INTERNATIONAL SEARCH REPORT**

International application No. **PCT/JP2023/031060**

**A. CLASSIFICATION OF SUBJECT MATTER**

***C07C 51/00***(2006.01)i; ***B01J 31/24***(2006.01)i; ***C07B 61/00***(2006.01)i; ***C07C 53/02***(2006.01)i; ***C07C 53/06***(2006.01)i
FI: C07C51/00; C07C53/06; C07B61/00 300; C07C53/02; B01J31/24 Z

According to International Patent Classification (IPC) or to both national classification and IPC

**B. FIELDS SEARCHED**

Minimum documentation searched (classification system followed by classification symbols)

C07C51/00; B01J31/00; C07B61/00; C07C53/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | CN 103113337 A (SUQIAN DEWEI CHEMICAL CO., LTD.) 22 May 2013 (2013-05-22) paragraph [0009], claim 2, examples 1-4 | 1-10, 15-19, 21-24 |
| Y | | 11-14, 20 |
| X | CHEN, Ruifang et al. Thermoregulated phase-transfer ligands and catalysis. Part VI. Two-phase hydroformylation of styrene catalyzed by the thermoregulated phase-transfer catalyst OPGPP/Rh. Journal of Organometallic Chemistry. 1998, 571(2), pages 201-204 abstract, pages 202-203, 2.1, 2.2, 3.1 | 1-10, 15-19, 21-24 |
| Y | | 11-14, 20 |
| X | CN 102352222 A (PEOP. REP. CHINA) 15 February 2012 (2012-02-15) claims 2-3, examples 1-3 | 1-10, 15-19, 21-24 |
| Y | | 11-14, 20 |
| X | CN 103613745 A (TIANJIN XIANGSHENG POWDER COATING CO., LTD.) 05 March 2014 (2014-03-05) claim 9, example 1 | 1-10, 15-19, 21-24 |
| Y | | 11-14, 20 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 November 2023** | **14 November 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
PCT/JP2023/031060

C. DOCUMENTS CONSIDERED TO BE RELEVANT

| Category* | Citation of document, with indication, where appropriate, of the relevant passages | Relevant to claim No. |
|---|---|---|
| Y | WO 2022/050234 A1 (NITTO DENKO CORP.) 10 March 2022 (2022-03-10) claims 1, 2, 11, 13, 14, synthesis examples 1-6 | 11-14, 20 |
| Y | WO 2022/050235 A1 (NITTO DENKO CORP.) 10 March 2022 (2022-03-10) claims 4-5, synthesis examples 1-2 | 11-14, 20 |

Form PCT/ISA/210 (second sheet) (January 2015)

**INTERNATIONAL SEARCH REPORT**
**Information on patent family members**

International application No.
**PCT/JP2023/031060**

| Patent document cited in search report | Publication date (day/month/year) | Patent family member(s) | Publication date (day/month/year) |
| --- | --- | --- | --- |
| CN 103113337 A | 22 May 2013 | (Family: none) | |
| CN 102352222 A | 15 February 2012 | (Family: none) | |
| CN 103613745 A | 05 March 2014 | (Family: none) | |
| WO 2022/050234 A1 | 10 March 2022 | EP 4209478 A1<br>claims 1, 2, 11, 13, 14, synthesis examples 1-6<br>CN 116096699 A | |
| WO 2022/050235 A1 | 10 March 2022 | EP 4209479 A1<br>claims 4-5, synthesis examples 1-2<br>CN 116096698 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5734286 B


### Non-patent literature cited in the description


- **E. PIDKO et al.** *ChemCatChem*, 2014, vol. 6, 1526-1530